# EUROPÄISCHE PATENTSCHRIFT

(11) **EP 1 200 437 B1**
(45) Veröffentlichungstag und Bekanntmachung des Hinweises auf die Patenterteilung: **10.03.2004**
(21) Anmeldenummer: 00956176.2
(22) Anmeldetag: 12.07.2000
(51) Int. Cl.: C07D 475/04

(54) **VERFAHREN ZUR HERSTELLUNG VON OPTISCH REINEN TETRAHYDROPTERINEN UND DERIVATEN, IM SPEZIELLEN OPTISCH REINER TETRAHYDROFOLSÄURE UND IHREN DERIVATEN, DURCH STEREOSPEZIFISCHE HYDRIERUNG**
METHOD FOR PRODUCING BY STEREOSPECIFIC HYDRATION TETRAHYDROPTERINS AND OPTICALLY PURE DERIVATIVES, IN PARTICULAR TETRAHYDROFOLIC ACID AND ITS OPTICALLY PURE DERIVATIVES
PROCEDE DE FABRICATION PAR HYDRATATION STEREOSPECIFIQUE DE TETRAHYDROPTERINES ET DE DERIVES OPTIQUEMENT PURS, NOTAMMENT D'ACIDE TETRAHYDROFOLIQUE ET DE SES DERIVES OPTIQUEMENT PURS

(30) Priorität: 14.07.1999 CH 130199
(43) Veröffentlichungstag der Anmeldung: 02.05.2002
(73) Patentinhaber: EPROVA AG Forschungsinstitut, CH-8200 Schaffhausen (CH)
(72) Erfinder: MÜLLER, Hans Rudolf, CH-8200 Schaffhausen (CH); MOSER, Rudolf, CH-8200 Schaffhausen (CH); GROEHN, Viola, CH-8212 Neuhausen am Rheinfall (CH); PUGIN, Benoît, CH-4142 Münchenstein (CH)
(86) Internationale Anmeldenummer: PCT/EP2000/006646
(87) Internationale Veröffentlichungsnummer: WO 2001/004120

(56) Entgegenhaltungen:
- EP-A- 0 256 982
- EP-A- 0 432 441
- EP-A- 0 537 842
- EP-A- 0 548 895
- EP-A- 0 551 642
- EP-A- 0 600 460
- EP-A- 0 682 026
- EP-A- 0 773 221
- BOYLE P.H. AND KEATING M.T.: 'Asymmetric Hydrogenation of a Carbon-Nitrogen Double Bond in Folic Acid' J.C.S. CHEM. COMM. 1974, Seiten 375 - 376

## Beschreibung

Die vorliegende Erfindung betrifft ein Verfahren zur Herstellung von Tetrahydropterin und Derivaten, besonders Tetrahydrofolsäure, Tetrahydrofolsäuresalzen, -estern und -estersalzen, durch Hydrierung von Pterin oder Pterinderivaten, besonders von Folsäure oder Folsäuresalzen, oder von Folsäureestern oder Folsäureestersalzen, in einem polaren Reaktionsmedium mit gelösten Metallkomplexen als Hydrierkatalysatoren. Die Erfindung betrifft ferner Additionssalze von Folsäureestern und Tetrahydrofolsäureestern.

Pterin entspricht der Formel 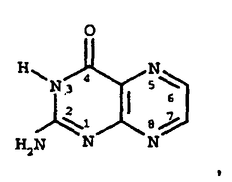 und es ist bekannt, dass Derivate dieser bi-heterocyclischen Verbindung in der Natur vorkommen und natürliche wie synthetische Derivate eine physiologische Wirksamkeit besitzen, wobei die Wirkung oft durch 5,6,7,8-Tetrahydropterine entfaltet wird. Es ist daher von Interesse, einen Zugang zu Tetrahydropterin und - derivaten als Zwischenprodukte oder physiologisch wirksame Verbindungen zu erschliessen. Ein bekanntes physiologisch wirksames Tetrahydropterinderivat ist Tetrahydrofolsäure, die unter anderem als Wachstumsfaktor der Leukozyten die Blutbildung beeinflusst. Die Tetrahydrofolsäure leitet sich von der Folsäure ab.

Folsäure entspricht der Formel I, 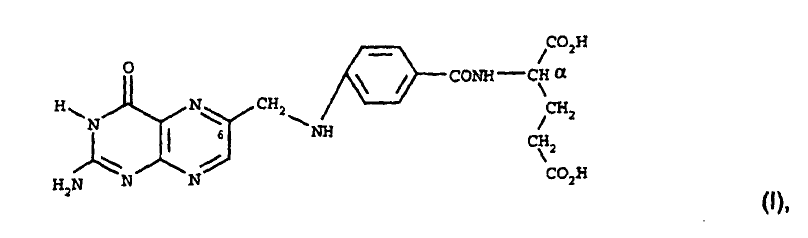 wobei das asymmetrische α-C-Atom im Glutaminsäurerest in der S-Konfiguration (αS) oder in der R-Konfiguration (αR)vorliegen kann. Die Enantiomeren der Folsäure werden nachfolgend als (αS)-Folsäure und (αR)-Folsäure bezeichnet. Das Gleiche gilt für die Folsäureester und ihre Derivate. Sie werden als (αS)-Folsäureester und (αR)-Folsäureester bezeichnet. Die natürlich vorkommende Folsäure entspricht der (αS)-Folsäure.

Tetrahydrofolsäure entspricht der Formel II, 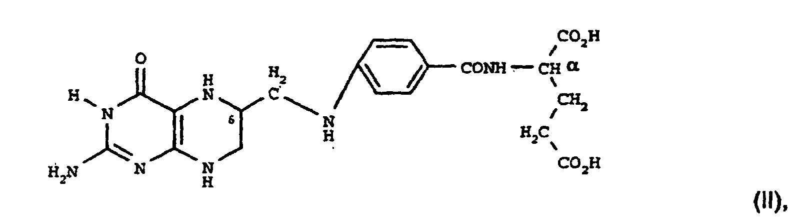 wobei das asymmetrische α-C-Atom im Glutaminsäurerest in der S-Konfiguration (αS) oder in der R-Konfiguration (αR) vorliegen kann und das asymmetrische C-Atom 6 im Tetrahydropterinrest in der R- (6R)- oder S-Konfiguration (6S) vorliegen kann. Die Diastereomeren der Tetrahydrofolsäure werden nachfolgend als (6S,αS)-, (6S,αR)-, (6R,αS)- und (6R,αR)-Tetrahydrofolsäure bezeichnet. Das Gleiche gilt für die Tetrahydrofofsäureester und ihre Derivate. Sie werden als (6S,αS)-, (6S,αR)-, (6R,aS)- und (6R,αR)-Tetrahydrofolsäureester bezeichnet. Die natürlich vorkommende Tetrahydrofolsäure entspricht der (6S,αS)-Tetrahydrofolsäure.

Im Folgenden beinhaltet die Bezeichnung Folsäure, Folsäureester und Folsäureestersalze, falls nicht anderweitig bezeichnet, immer die beiden Enantiomeren (αS) und (αR) und die Bezeichnung Tetrahydrofolsäure, Tetrahydrofolsäureester und Tetrahydrofolsäureestersalze alle möglichen Diastereomeren. Die Bezeichnung Folsäureestersalze und Tetrahydrofolsäureestersalze umfasst im Rahmen der Erfindung Additionssalze von Folsäureestern und Tetrahydrofolsäureestern mit Säuren.

Tetrahydrofolsäure hat in Form von 5-Formyl- oder 5-Methylderivaten und ihren physiologisch verträglichen Salzen eine breite therapeutische Anwendung gefunden. Es ist seit langem bekannt, dass sich die biologische Aktivität der natürlich vorkommenden und der in der Natur nicht vorkommenden Diastereomeren der reduzierten Folate, zum Beispiel des natürlichen (6S,αS)-Diastereomeren der Tetrahydrofolsäure und des unnatürlichen (6R,αS)-Diastereomeren der Tetrahydrofolsäure, stark unterscheidet. Es ist daher zweckmässig, therapeutische Präparate bereitzustellen, in dem nur die aktivste Form enthalten oder diese zumindest hoch angereichert ist.

Tetrahydrofolsäure wird industriell im Allgemeinen durch heterogene Hydrierung der beiden Imingruppen im Pterinsystem von (αS)-Folsäure hergestellt, wobei man üblicherweise ein äquimolares Gemisch aus zwei Diastereomeren erhält, das heisst aus (6S,αS)-Tetrahydrofolsäure und (6R,αS)-Tetrahydrofolsäure. Das äquimolare Gemisch kann für pharmazeutische Zubereitungen verwendet werden. Man kann aber auch zuvor das gewünschte Diastereomere der Tetrahydrofolsäure durch fraktionierte Kristallisation anreichern oder in reiner Form gewinnen, wofür verschiedene Verfahren bekannt sind, siehe zum Beispiel EP-0 495 204. Dieses Verfahren kann aus oekonomischer Sicht insofern nicht überzeugen, als dann von vornherein das unerwünschte Diastereomere anderweitig verwendet werden muss.

Um diesen Substanzverlust zu erniedrigen oder gar zu vermeiden, sind auch schon diastereoselektive (asymmetrische) Hydrierungen der Folsäure vorgeschlagen worden. So wird in der EP-0 551 642 beschrieben, auf einem Träger immobilisierte Rh(I)-Komplexe mit optisch aktiven Diphosphinen zur Hydrierung von Folsäure in wässriger Pufferlösung zu verwenden. Die optischen Ausbeuten erreichen bis zu etwa 50% de, wobei aber berücksichtigt werden muss, dass diese Werte durch die Derivatisierung vor Bestimmung der optischen Ausbeute beeinflusst sein können und nicht den effektiven Werten nach der Hydrierung entsprechen müssen. So werden selbst vom Erfinder die in EP-0 551 642 angegebenen Werte in Zweifel gezogen (siehe dazu H. Brunner et al. in Chem. Ber./Receuil, 1997, No. 130, Seiten 55-61, im Speziellen Seite 56, rechte Spalte, 1. Abschnitt). Nachteilig bei dieser heterogenen Hydrierung ist die auf den Einfluss des Trägermaterials zurückzuführende starke Schwankung der Diastereoselektivität, was die Reproduzierbarkeit erheblich beeinflusst. Femer müssen niedrige Verhältnisse von Substrat zu Katalysator (hohe Katalysatormengen) angesetzt werden, weil bei einem Substrat/Katalysator-Verhältnis von > 40 sowohl die chemische Ausbeute als auch die optische Ausbeute drastisch absinken. Die Abtrennung, Reinigung und der Wiedereinsatz des Katalysators führt ebenfalls zu einer Verschlechterung von chemischer und optischer Ausbeute. Ein besonderer Nachteil ist die geringe Katalysatoraktivität, so dass trotz hoher Katalysatorkonzentrationen relativ lange Reaktionszeiten benötigt werden. Das Verfahren ist daher nicht für den industriellen Massstab geeignet.

Aus den EP-0 256 982, EP-0 564 406 und EP-0 646 590 ist es bekannt, dass man Iridiummetallkomplexe mit chiralen Diphosphinliganden für die stereoselektive Hydrierung von prochiralen Iminen verwenden kann. Die Hydrierung von Imingruppen, die Teil eines aromatischen Ringsystems sind, ist jedoch nicht offenbart.

In der EP-A-0 773 221 werden kristalline Magnesium- und Calciumsalze der 6(R,S)-, 6(S)- und 6(R)-Tetrahydrofolsäure und ein Kristallisationsverfahren zu deren Herstellung beschrieben. Die EP-A-0 682 026 offenbart kristalline 6(S)- und 6(R)-Tetrahydrofolsäure und ein Kristallisationsverfahren zu deren Herstellung. Die EP-A-0 600 460 beschreibt ein Verfahren zur Herstellung von 6(S)-Tetrahydrofotsäure aus Alkalimetallsalzen des Diastereomerengemisches durch Zugabe von Säure. Die EP-A-548 895 offenbart die Herstellung von (6R)-N(5),N(10)-Methenyl-5,6,7,8-Tetrahydrofolsäuresalzen durch Behandlung von Ammonium- oder Alkalimetallsalzen von (6RS)-N(10)-Formyl-5,6,7,8-tetrahydrofolsäure in wässriger Pufferlösung mit Säuren. In der EP-A-0 537 842 wird die Hydrierung von Folsäure mit einem Überschuss an NaBH₄ zu (6RS)-N⁵-Formyl-5,6,7,8-tetrahydrofolsäure und (6RS)-N⁵-Methyl-5,6,7,8-tetrahydrofolsäure beschrieben. Die EP-A-0 432 441 offenbart eine enzymatische Umwandlung eines Diastereomeren des Racemats von Tetrahydrofolsäure in 10-Formyl-5,6S,7,8-Tetrahydrofolsäure.

P. H. Boyle et al. beschreiben in Tetrahedron Vol. 44, No. 16 (1988) 5179-5188, dass bei der Hydrierung von Folsäuresilylestern mit einem asymmetrischen Rhodium/Diphosphin-komplex in benzolischer Lösung selbst in Gegenwart von Wasser keinerlei Wasserstoffaufnahme erfolgt und das Substrat unverändert zurückgewonnen wird.

P. H. Boyle et al. beschreiben in J.C.S. Chem. Comm. (1974), Seiten 375-376 eine katalytische Hydrierung von Folsäure mit Wasserstoff, bei dem eine neutralisierte NaOH-Lösung der Folsäure zu einem Rh-Katalysator gegeben wird, der durch Zugabe von NaBH₄ und (+)- oder (-)-N-1-Phenylethylformamid zu (Pyridin)₃RhCl₃ erhalten wird. Vor der Aufarbeitung des Reaktionsgemisches muss 2-Mercaptoethanol zugegeben werden. Die Ausbeute an biologisch aktiver Tetrahydrofolsäure ist sehr niedrig.

Hydrierungen von Pterin und Pterinderivaten, wie zum Beispiel Folsäure, mit Wasserstoff in einem Reaktionsmedium und darin gelösten Hydrierkatalysatoren in Form von Metallkomplexen sind noch nicht bekannt, obwohl ein technischer Bedarf für ein solches Verfahren besteht.

Es wurde nun überraschend gefunden, dass man die Imingruppen im aromatischen Pterinsystem, insbesondere der Folsäure und der Folsäureester, in Gegenwart gelöster Metallkomplexe als Hydrierkatalysatoren mit Wasserstoff hydrieren kann, wenn man polare Reaktionsmedien verwendet, zum Beispiel ein wässriges oder ein alkoholisches Reaktionsmedium. Das Verfahren zeichnet sich durch überraschend kurze Reaktionszeiten bei erhöhten Umsätzen aus, was auf die hohe Katalysatoraktivität und -produktivität hinweist, die selbst bei erhöhten Verhältnissen von Substrat zu Katalysator beobachtet werden. Das Verfahren ist wirtschaftlich und reproduzierbar und eignet sich auch für einen industriellen Massstab.

Es wurde ferner überraschend gefunden, dass man unter diesen Reaktionsbedingungen selbst asymmetrische Hydrierungen durchführen kann und sogar hohe optische Ausbeuten erzielt werden, die über 50% ee oder de liegen können, wenn man als Hydrierkatalysatoren Metallkomplexe mit chiralen Liganden verwendet. Mit Hilfe einer asymmetrischen Hydrierung kann man zum Beispiel aus (αS)-Folsäure oder (αS)-Folsäureestern beziehungsweise (αS)-Folsäureestersalzen je nach optischer Induktion des Liganden Gemische von Diastereomeren erhalten, in denen das (6R,αS)- beziehungsweise (6S,αS)-Diastereomere überwiegt. Geht man von (αR)-Folsäure oder (αR)-Folsäureestern beziehungsweise (αR)-Folsäureestersalzen aus, so erhält man Gemische, in denen das (6R,αR)-beziehungsweise (6S,αR)-Diastereomere überwiegt.

Ein erster Gegenstand der Erfindung ist ein Verfahren zur Herstellung von Tetrahydropterin und Tetrahydropterinderivaten durch Hydrierung von Pterin und Pterinderivaten mit Wasserstoff in Gegenwart eines Hydrierkatalysators, das dadurch gekennzeichnet ist, dass man die Hydrierung in einem polaren Reaktionsmedium durchführt und in dem Reaktionsmedium lösliche d-8-Metallkomplexe als Hydrierkatalysatoren verwendet, die tertiäre Phosphine. tertiäre Phosphane als Liganden, bidentate Liganden mit tertiären Aminogruppen und tertiären Phosphingruppen, oder bidentate Liganden mit zwei tertiären Phosphingruppen enthalten, wobei bidentate Liganden mit dem Metallatom einen 5- bis 10-gliedrigen Ring bilden.

Die Hydrierung kann über Dihydropterinzwischenstufen verlaufen. Im Rahmen der Erfindung wird auch die Verwendung solcher Zwischenstufen, beziehungsweise Dihydropterinen und Dihydropterinderivaten als Ausgangsverbindungen zur Hydrierung umfasst. Bei diesen Ausgangsverbindungen kann es sich um sämtliche Tautomeren handeln, zum Beispiel um 5,6-, 7,8- und 5,8-Dihydropterine und Dihydropterinderivate, und auch um Enamine (6-Aminoethenyl-tetrahydropterine und -derivate).

Polares Reaktionsmedium bedeutet im Rahmen der Erfindung bevorzugt ein wässriges oder alkoholisches Reaktionsmedium.

Ein bevorzugter Gegenstand der Erfindung ist ein Verfahren zur Herstellung von Tetrahydrofolsäure, Tetrahydrofolsäuresalzen, Tetrahydrofolsäureestern oder Tetrahydrofolsäureestersalzen durch Hydrierung von Folsäure, Folsäuresalzen, Folsäureestern oder Folsäureestersalzen mit Wasserstoff in Gegenwart eines Hydrierkatalysators, das dadurch gekennzeichnet ist, dass man die Hydrierung bei erhöhtem Druck in Gegenwart von im Reaktionsmedium gelösten Metallkomplexen als Hydrierkatalysatoren durchführt, mit der Massgabe, dass bei Verwendung von Folsäure und deren Carbonsäuresalzen ein wässriges, sowie bei Verwendung von Folsäureestern und Folsäureestersalzen ein alkoholisches Reaktionsmedium vorliegt.

Ein weiterer bevorzugter Gegenstand der Erfindung ist ein Verfahren zur Herstellung von chiralen Tetrahydropterinderivaten durch asymmetrische Hydrierung von prochiralen Pterinderivaten mit Wasserstoff in Gegenwart eines Hydrierkatalysators, das dadurch gekennzeichnet ist, dass man die Hydrierung in einem polaren Reaktionsmedium durchführt und in dem Reaktionsmedium lösliche Metallkomplexe als Hydrierkatalysatoren verwendet, wobei die Metallkomplexe chirale Liganden enthalten. Prochirale Pterinderivate für die asymmetrische Hydrierung sind hauptsächlich in 6-, 7-, oder in 6- und 7-Stellung substituierte Pterine.

Ein anderer bevorzugter Gegenstand der Erfindung ist ein Verfahren zur Herstellung von chiraler Tetrahydrofolsäure, chiralen Tetrahydrofolsäuresalzen, Tetrahydrofolsäureestern oder Tetrahydrofolsäureestersalzen durch asymmetrische Hydrierung von Folsäure, Folsäuresalzen, Folsäureestern oder Folsäureestersalzen mit Wasserstoff in Gegenwart eines Hydrierkatalysators, das dadurch gekennzeichnet ist, dass man die Hydrierung bei erhöhtem Druck in Gegenwart von im Reaktionsmedium gelösten Metallkomplexen als Hydrierkatalysatoren durchführt, wobei die Metallkomplexe chirale Liganden enthalten, mit der Massgabe, dass bei Verwendung von Folsäure und deren Carbonsäuresalzen ein wässriges, sowie bei Verwendung von Folsäureestern und Folsäureestersalzen ein alkoholisches Reaktionsmedium vorliegt.

Wenn man (αS)- oder (αR)-Folsäure oder deren Carbonsäuresalze, Folsäureester oder Folsäureestersalze als Ausgangsprodukt für die Hydrierung einsetzt, enthalten die Reaktionsprodukte je nach optischer Induktion durch den Liganden im Metallkomplex einen Überschuss der (6S,αS)- oder (6R,αS), bzw. (6S,αR)- oder (6R,αR)- Diastereomeren. Wenn man ein äquimolares Gemisch der (αS)- und (αR)-Folsäure oder deren Carbonsäuresalze, Folsäureester und Folsäureestersalze einsetzt, enthalten die Reaktionsprodukte je nach optischer Induktion durch den Liganden im Metallkomplex entweder einen Überschuss der (6R,αS), (6R,αR)- oder der (6S,αS), (6S,αR)-Diastereomeren.

Optischer Überschuss bei der asymmetrischen Hydrierung bedeutet im Rahmen der Erfindung, dass im Gemisch der Diastereomeren ein Diastereomer oder ein Diastereomerenpaar überwiegt. Bevorzugt beträgt das Verhältnis des einen zu dem anderen Diastereomeren oder Diastereomerenpaar mindestens 55:45, besonders bevorzugt mindestens 60:40, und insbesondere bevorzugt mindestens 75:25.

Pterin und prochirale Pterine sind bekannt oder nach bekannten oder analogen Verfahren herstellbar. Prochirale Pterine sind entweder in der 6- oder der 7-, oder in der 6- und 7-Stellung substituiert. Prochirale Pterine können der Formel A entsprechen, 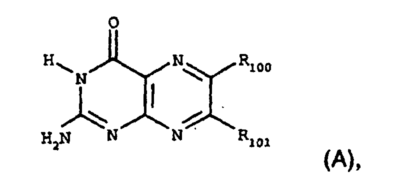 worin R₁₀₁ H ist oder unabhängig die Bedeutung von R₁₀₀ hat, und R₁₀₀ einen über ein C-, O- oder N-Atom gebundenen organischen Rest mit 1 bis 50 C-Atomen darstellt, der nicht unterbrochen oder der durch eine oder mehrere Gruppen ausgewählt aus -O-, -NH-, -N(C₁-C₄Alkyl)-, -C(O)-, -C(O)O-, -OC(O)-, -OC(O)O-, - C(O)NH-, -NHC(O)-, -NHC(O)O-, -OC(O)NH-, -NHC(O)NH-, -C(O)N(C₁-C₄-Alkyl)-, -N(C₁-C₄-Alkyl)C(O)-, -N(C₁-C₄-Alkyl)C(O)O-, -OC(O)N(C₁-C₄-Alkyl)-, - N(C₁-C₄-Alkyl)C(O)N(C₁-C₄-Alkyl)- unterbrochen ist, und der unsubstituiert oder mit F, Cl, Br, -CN, -OCN, -NCO, -OH, -NH₂, -NHC₁-C₄-Alkyl, -N(C₁-C₄-Alkyl)₂, C₁-C₄-Alkyl, C₁-C₄-Halogenalkyl, C₁-C₄-Hydroxyalkyl, C₁-C₄-Alkoxy, C₁-C₄.Halogenalkoxy, -C(O)OH, -C(O)OM₁₀₀, -C(O)OC₁-C₄-Alkyl, -C(O)NH₂, - C(O)NHC₁-C₄-Alkyl, -C(O)N(C₁-C₄-Alkyl)₂, R₁₀₂-C(O)O-, R₁₀₂-OC(O)O-, R₁₀₂.C(O)NH-, R₁₀₂-C(O)N(C₁-C₄-Alkyl)-, R₁₀₂-NHC(O)NH-, R₁₀₃C(O)- oder -CH(O) substituiert ist,
M₁₀₀ für Li, K, Na, NH₄⁺, oder Ammonium mit 1 bis 16 C-Atomen steht,
R₁₀₂ für C₁-C₈-Alkyl, C₅- oder C₆-Cycloalkyl, Phenyl oder Benzyl steht, und
R₁₀₃ C₁-C₄-Alkyl, Phenyl oder Benzyl bedeutet.

R₁₀₀ enthält als organischen Rest bevorzugt 1 bis 30 C-Atome, besonders bevorzugt 1 bis 20 C-Atome, und insbesondere bevorzugt 1 bis 12 C-Atome, und gegebenenfalls wenigstens 1 Heteroatom ausgewählt aus der Gruppe O, N und P. Beispiele für organische Reste sind Alkyl, Cycloalkyl, Cycloalkylalkyl, Phenyl, Naphthyl, Phenylalkyl und Naphthylalkyl, sowie entsprechende Heteroreste mit Heteroatomen ausgewählt aus der Gruppe O und N.

Die C₁-C₄-Alkylgruppe bedeutet bevorzugt Methyl oder Ethyl. R₁₀₁ steht bevorzugt für H. M₁₀₀ als Ammonium mit 1 bis 16 C-Atomen kann zum Beispiel H₃N(C₁-C₄-Alkyl)+, H₂N(C₁-C₄-Alkyl)₂⁺, HN(C₁-C₄-Alkyl)₃⁺ oder N(C₁-C₄-Alkyl)4⁺ sein; wobei Alkyl bevorzugt Methyl, Ethyl oder n-Butyl ist. R₁₀₂ enthält als Alkyl bevorzugt 1 bis 4 C-Atome und kann zum Beispiel Methyl, Ethyl, Propyl und Butyl sein. R₁₀₃ bedeutet bevorzugt Methyl, Ethyl oder Phenyl.

Eine bevorzugte Untergruppe von Verbindungen der Formel A sind solche, worin R₁₀₁ für H steht und R₁₀₀ -CH3, Phenyl, -CH=O, gegebenenfalls mit Acetyl, Trifluoracetyl oder =O substituiertes C₂-C₆-Mono- oder Polyhydroxyalkyl, - C(O)-C₁-C₄-Alkyl, -C(O)OH, C(O)OC₁-C₄-Alkyl, -C(O)NH₂, -C(O)NHC₁-C₄-Alkyl, -C(O)N(C₁-C₄-Alkyl)₂, -CH₂(CH₂)_{0,1}-OH, -CH₂(CH₂)_{0,1}-NH₂, -CH₂(CH₂)_{0,1}-NHC₁-C₄-Alkyl, oder -CH(R₁₀₄)-(R₁₀₅)-p-C₆H₄-C(O)-R₁₀₆ bedeutet, R₁₀₄ H, Methyl oder Ethyl darstellt,
R₁₀₅ eine direkte Bindung, -CH₂-, -O-, -NH-, -NCH₃-, -N[HC(O)]-, -N[CH₃C(O)]-, -N[CF₃C(O)]-, -NHC(O)-, oder -OC(O)- bedeutet, und
R₁₀₆ für -OH, -NH₂, -NHCH₃, -N(CH₃)₂, oder -NHR₁₀₇ steht, worin R₁₀₇ ein über ein α-Kohlenstoffatom gebundener Rest einer natürlichen oder unnatürlichen Aminosäure oder eines Peptids aus natürlichen oder unnatürlichen Aminosäuren mit 2 bis 12 Aminosäureeinheiten ist.

Das Mono- oder Polyhydroxyalkyl enthält bevorzugt 2 bis 4 C-Atome und bevorzugt 1 bis 4 an verschiedene C-Atome gebundene OH-Gruppen.

Einige Beispiele für R₁₀₀ in Formel A sind -CHO, -C(O)-CH₃, -CH₂-NH₂, -CH₂CH₂-NH₂, -CH₂-OH, -CH₂CH₂-OH, -C(O)-OH, -CH₂-C(O)-OH, -C(O)-NH₂, -CH₂-C(O)-NH₂, -CH₂-NH-p-C₆H₄-C(O)OH (bei R₁₀₁ gleich H = Pteroinsäure), -CH₂CH₂-NH-p-C₆H₄-C(O)OH, -CH₂CH₂-NH-p-C₆H₄-C(O)-NH-CH(CO₂H)-CH₂CH₂-C(O)OH (bei R₁₀₁ gleich H = Homofolsäure), -C(O)-CH(OH)-CH₃, Biopterine bei R₁₀₁ gleich H und R₁₀₀ gleich -CH(OH)-CH(OH)-CH₃, und Neopterine bei R₁₀₁ gleich H und R₁₀₀ gleich -CH(OH)-CH(OH)-CH₂-OH, -CH₂-N(CHO)-p-C₆H₄-C(O)-NH-CH(CO₂H)-CH₂CH₂-C(O)OH (bei R₁₀₁ gleich H = 10-Formylfolsäure), sowie -CH₂-NH-p-C₆H₄-C(O)-NH-CH(CO₂H)-CH₂CH₂-C(O)OH (bei R₁₀₁ gleich H = Folsäure). Die chiralen C-Atome der Biopterine und Neopterine können als Racemate oder optische lsomere vorliegen, zum Beispiel

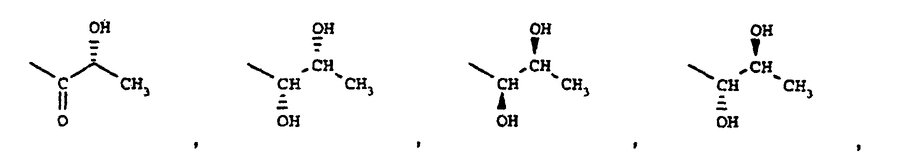

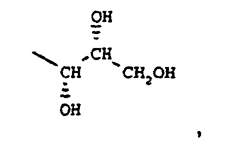

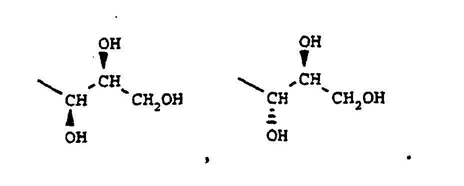

Metallkomplexe als lösliche Hydrierkatalysatoren enthalten im wesentlichen d-8 Metalle; besonders bevorzugt d-8 Metalle ausgewählt aus der Gruppe Rhodium (Rh), Iridium (Ir) und Ruthenium (Ru).

Liganden für Metalle als lösliche Hydrierkatalysatoren enthalten häufig tertiäre Amin- und/oder Phosphingruppen als komplexbildende Gruppen, wobei die Liganden mit dem Metallatom einen 5- bis 10-, und bevorzugt 5- bis 7-gliedrigen Ring bilden. Bevorzugt sind Liganden, die eine tertiäre Amingruppe und eine tertiäre Phosphingruppe oder zwei tertiäre Phosphingruppen enthalten.

Besonders bevorzugt sind organische achirale oder chirale ditertiäre Diphosphinliganden. Chirale ditertiäre Diphosphinliganden bedeutet im Rahmen der Erfindung, dass das Diphosphin wenigstens ein chirales Element aufweist und wenigstens zwei optische Isomere umfasst. Die optische Isomerie kann zum Beispiel durch stereogene Zentren (asymmetrische C-Atome), Atropisomerie oder planare Chiralität bedingt sein. Stereogene Zentren können in den Phosphinsubstituenten und/oder im Gerüst und/oder Seitengruppen des Gerüsts des Diphosphins vorhanden sein. Durch die Auswahl der Enantiomeren oder Diastereomeren von Liganden kann die optische Induktion gesteuert bzw. umgekehrt werden. Wenn diese nicht vorhergesagt werden kann, kann die optische Induktion durch einen einfachen Testversuch ermittelt werden. Die Hydrierung von Folsäureestersalzen mit dem Katalysator Rh/(R)-BINAP führt zum Beispiel zu einer Anreicherung des (6S,αS)-Diastereomeren des Tetrahydrofolsäuredimethylestersalzes. Wird die gleiche Hydrierung mit dem Katalysator Rh/(S)-BINAP durchgeführt, so erhält man eine gleich hohe Anreicherung des (6R,αS)-Diastereomeren des Tetrahydrofolsäuredimethylestersalzes.

Folsäure kann als reine (αS)- oder (αR)-Folsäure oder in jedem beliebigen Mischungsverhältnis beider Enantiomeren eingesetzt werden. Entsprechende Folsäureester sind durch übliche Veresterungsverfahren erhältlich. Die Folsäureester können die gleichen Kohlenwasserstoffreste oder Heterokohlenwasserstoffreste in der Estergruppe enthalten, wie es nachfolgend für die Verbindungen der Formel III beschrieben ist, einschliesslich der Bevorzugungen. Bevorzugt sind (αS)-Folsäure und (αS)-Folsäureester.

Die Folsäure kann auch in Form ihrer Carbonsäuresalze vorliegen. Geeignet sind zum Beispiel Alkalimetall- und Erdalkalimetall- sowie Ammoniumsalze. Unter den Alkalimetall- und Erdalkalimetallsalzen sind die Natrium-, Kalium-, Magnesiumund Calciumsalze bevorzugt. Unter den Ammoniumsalzen sind NH₄⁺ und die Kationen von primären, sekundären und tertiären Aminen sowie quartemäres Ammonium geeignet. Die Amine können zum Beispiel 1 bis 30 C-Atome, bevorzugt 1 bis 24 C-Atome, und das quarternäre Ammonium kann zum Beispiel 4 bis 40, und bevorzugt 4 bis 32 C-Atome aufweisen. Einige Beispiele für Ammonium sind Methyl-, Ethyl-, n-Propyl-, n-Butyl, n-Hexyl-, n-Octyl-, Phenyl-, Benzyl-, Dimethyl-, Diethyl-, Di-n-propyl-, Di-n-butyl-, Di-n-hexyl-, Di-n-octyl-, Methyl-ethyl-, Methyl-n-butyl-, Methyl-n-octyl-, Tetra- oder Pentamethylen-, Trimethyl-, Triethyl-, Tri-n-butyl-, Trin-octyl-, Tetramethyl-, Tetra-n-butyl-, Tetra-n-octyl- und trimethyln-octyl-ammonium. Die Amingruppen der Folsäuresalze können zusätzlich auch mit ein- bis dreibasischen anorganischen oder organischen Säuren ein Salz bilden, und die Gruppe x HA enthalten, wobei x und HA die nachfolgend für Folsäureestersalze der Formel III angegebenen Bedeutungen hat, einschliesslich der Bevorzugungen.

Die Folsäureestersalze in Form ihrer Enantiomeren oder deren Mischungen können der Formel III entsprechen, 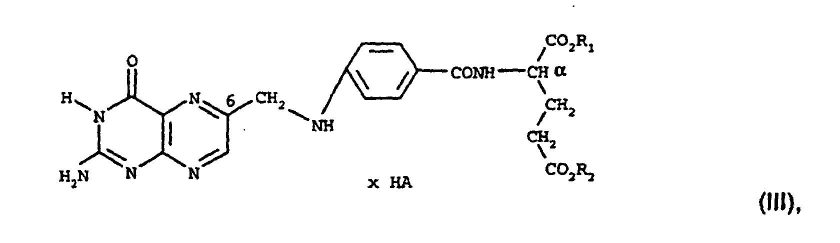 worin R₁ oder R₂ H sind, und eines von R₁ oder R₂, oder beide R₁ und R₂ unabhängig voneinander einen monovalenten Kohlenwasserstoffrest oder einen über ein C-Atom gebundenen Heterokohlenwasserstoffrest mit Heteroatomen ausgewählt aus der Gruppe -O-, -S- und -N- darstellen,
HA für eine ein- bis dreibasische anorganische oder organische Säure steht und x eine ganze Zahl von 1 bis 6 oder gebrochene Zahl zwischen 0 und 6 bedeutet.

R₁ und R₂ können unabhängig voneinander gewählt werden, bevorzugt sind sie aber identisch. Bevorzugt stellen R₁ und R₂ einen Kohlenwasserstoffrest dar. Bei R₁ und R₂ als Kohlenwasserstoffrest kann es sich um aliphatische Reste mit 1 bis 20, bevorzugt 1 bis 12, besonders bevorzugt 1 bis 8, und insbesondere bevorzugt 1 bis 4 C-Atome; um cycloaliphatische oder cycloaliphatisch-aliphatische Reste mit 3 bis 8 Ringkohlenstoffatomen und 1 bis 6 C-Atomen im aliphatischen Rest, um aromatische Kohlenwasserstoffreste mit 6 bis 14 C-Atomen, besonders bevorzugt 6 bis 10 C-Atomen, oder um aromatisch-aliphatische Reste mit 7 bis 15 C-Atomen, besonders bevorzugt 7 bis 10 C-Atomen handeln.

Bei dem Heterokohlenwasserstoffrest kann es sich um Heteroalkyl mit 2 bis 16 C-Atomen, bevorzugt 2 bis 10 C-Atomen, und besonders bevorzugt 2 bis 6 C-Atomen; um heterocycloaliphatische Reste mit 3 bis 8, bevorzugt 5 oder 6 Ringgliedern; um heterocycloaliphatisch-aliphatische Reste mit 3 bis 8, bevorzugt 5 oder 6 Ringgliedern, und 1 bis 6, bevorzugt 1 bis 4 C-Atomen im aliphatischen Rest; um heteroaromatische Reste mit bevorzugt 4 bis 13 C-Atomen, und besonders bevorzugt 4 bis 9 C-Atomen und wenigstens einem Heteroatom; und um heteroaromatisch-aliphatische Reste mit bevorzugt 4 bis 13 C-Atomen, und besonders bevorzugt 4 bis 9 C-Atomen und wenigstens einem Heteroatom, und 1 bis 6, bevorzugt 1 bis 4 C-Atomen im aliphatischen Rest handeln; wobei die Heteroreste wenigstens ein Heteroatom ausgewählt aus der Gruppe -O-, -S- und - N- und bevorzugt -O- und
- N- enthalten.

Die Kohlenwasserstoffreste können zum Beispiel ausgewählt sein aus der Gruppe lineares und verzweigtes C₁-C₂₀-Alkyl, C₃-C₈- und bevorzugt C₄-C₇-Cycloalkyl, C₃-C₈-Cycloalkyl-C₁-C₆-Alkyl und bevorzugt C₄-C₇-Cycloalkyl-C₁-C₄-Alkyl, C₆-C₁₀-Aryl oder C₇-C₁₂-Aralkyl.

Die Heterokohlenwasserstoffreste können zum Beispiel ausgewählt sein aus der Gruppe C₂-C₁₆-Heteroalkyl, C₂-C₇- und bevorzugt C₄-C₅-Heterocycloalkyl, C₄-C₇und bevorzugt C₄-C₅-Heterocycloalkyl-C₁-C₆-Alkyl , C₄-C₉- und bevorzugt C₄-C₅-Heteroaryl, und C₅-C₁₂- und bevorzugt C₅-C₁₀-Heteroaralkyl, wobei die Heteroreste 1 bis 3 und bevorzugt 1 oder 2 Heteroatome aus der Gruppe -O- und -N- enthalten.

R₁ und R₂ können lineares oder verzweigtes Alkyl sein, das bevorzugt 1 bis 12, bevorzugter 1 bis 8, und besonders bevorzugt 1 bis 4 C-Atome enthält. Beispiele sind Methyl, Ethyl, und die Isomeren von Propyl, Butyl, Pentyl, Hexyl, Heptyl, Octyl, Decyl, Dodecyl, Tetradecyl, Hexadecyl, Octadecyl und Eicosyl. Bevorzugt ist das Alkyl linear und bevorzugt ist das Alkyl Methyl, Ethyl, n-Propyl und n-Butyl.

R₁ und R₂ enthalten als Cycloalkyl bevorzugt 4 bis 7 und besonders bevorzugt 5 oder 6 Ringkohlenstoffatome. Beispiele für Cycloalkyl sind Cyclopropyl, Cyclobutyl, Cyclopentyl, Cyclohexyl, Cycloheptyl und Cyclooctyl. Besonders bevorzugt ist Cyclohexyl.

R₁ und R₂ enthalten als Cycloalkyl-alkyl bevorzugt 4 bis 7 und besonders bevorzugt 5 oder 6 Ringkohlenstoffatome, und bevorzugt 1 bis 4 und besonders bevorzugt 1 oder 2 C-Atome im aliphatischen Rest. Beispiele für Cycloalkyl-alkyl sind Cyclopropylmethyl oder -ethyl, Cyclobutylmethyl oder -propyl, Cyclopentylmethyl oder -ethyl, Cyclohexylmethyl oder -ethyl, Cycloheptylmethyl und Cyclooctylmethyl. Besonders bevorzugt ist Cyclohexylmethyl oder -ethyl.

R₁ und R₂ können als Aryl für Naphthyl und bevorzugt Phenyl stehen. R₁ und R₂ sind als Aralkyl bevorzugt Phenylalkyl mit bevorzugt 1 bis 4 C-Atomen im Alkyl. Beispiele sind Benzyl und β-Phenylethyl.

R₁ und R₂ können als Heteroalkyl zum Beispiel C₁-C₄-Alkyl-X₁-C₂-C₄-alkyl sein, worin X₁ für O oder NC₁-C₄-Alkyl steht. Beispiele sind Methoxyethyl und Ethoxyethyl.

R₁ und R₂ können als Heterocycloalkyl zum Beispiel Pyrrolidinyl, Piperidinyl, Morpholinyl, Tetrahydropyranyl oder Piperazinyl sein.

R₁ und R₂ können als Heterocycloalkyl-alkyl zum Beispiel Pyrrolidinylmethyl oder -ethyl, Piperidinylmethyl oder -ethyl, Morpholinylmethyl oder -ethyl, Tetrahydropyranylmethyl oder -ethyl, oder Piperazinylmethyl oder -ethyl sein.

R₁ und R₂ können als Heteroaryl zum Beispiel Thiophenyl, Furanyl, Pyranyl, Pyrrolyl, Imidazolyl, Pyridinyl, Pyrimidinyl, Pyrazinyl, Indolyl, Chinolinyl, Oxazolyl oder Isooxazolyl sein.

R₁ und R₂ können als Heteroaralkyl zum Beispiel Furanylmethyl oder -ethyl, Pyranylmethyl oder -ethyl, Pyrrolylmethyl oder -ethyl, Imidazolylmethyl oder -ethyl, Pyridinylmethyl oder -ethyl, Pyrimidinylmethyl oder -ethyl, Pyrazinylmethyl oder - ethyl, Indolylmethyl oder -ethyl, Chinolinylmethyl oder -ethyl sein.

Eine bevorzugte Gruppe von Verbindungen der Formel III sind solche, worin R₁ und R₂ unabhängig voneinander C₁-C₄-Alkyl, C₅- oder C₆-Cycloalkyl, Phenyl, C₁-C₄-Alkylphenyl, Benzyl oder C₁-C₄-Alkylbenzyl darstellen. Bevorzugt sind R₁ und R₂ gleiche Reste. Ganz besonders bevorzugt stellen R₁ und R₂ C₁-C₄-Alkyl dar, zum Beispiel Methyl oder Ethyl.

In Formel III bedeutet x bevorzugt eine ganze Zahl von 1 bis 4 oder eine gebrochene Zahl zwischen 0,2 und 4, besonders bevorzugt eine ganze Zahl von 1 bis 3 oder eine gebrochene Zahl zwischen 0,5 und 3, und ganz besonders bevorzugt 1 oder 2 oder eine gebrochene Zahl zwischen 0,5 und 2.

Wenn sich die Säure HA in Formel III von einer anorganischen Säure ableitet, so kann es sich zum Beispiel um HCl, HBr, HI, H₂SO₃, H₂SO₄, H₂CO₃, HNO₃, H₃PO₃, H₃PO₄, HBF₄ oder H₂PF₆ handeln.

HA in Formel III stellt bevorzugt eine organische Säure dar. Die organischen Säuren leiten sich bevorzugt von Carbonsäuren, Sulfonsäuren, und Phosphonsäuren ab, die 1 bis 18, bevorzugt 1 bis 12, und besonders bevorzugt 1 bis 8 C-Atome enthalten.

Die organischen Säuren entsprechen bevorzugt der Formel IV,

R₃-X₂-OH (IV),

worin X₂ für -C(O)-, -S(O)₂- oder -P(O)OH- steht, und
R₃ unsubstituiertes oder mit Halogen, besonders Fluor oder Chlor, Hydroxyl, Carboxyl, Nitril, C₁-C₄-Alkyl, C₁-C₄-Alkoxy oder C₁-C₄-Halogenalkyl substituiertes, lineares oder verzweigtes C₁-C₁₈- und bevorzugt C₁-C₁₂-Alkyl, C₃-C8- und bevorzugt C₄-C₇-Cycloalkyl, C₃-C₈- und bevorzugt C₄-C₇-Cycloalkyl-C₁-C₄-Alkyl, C₆-C₁₀-Aryl oder C₇-C₁₂-Aralkyl bedeutet.

R₃ kann lineares oder verzweigtes Alkyl sein, das bevorzugt und besonders bevorzugt 1 bis 4 C-Atome enthält. Beispiele sind Methyl, Ethyl, und die Isomeren von Propyl, Butyl, Pentyl, Hexyl, Heptyl, Octyl, Decyl, Dodecyl, Tetradecyl, Hexadecyl, Octadecyl und Eicosyl. Bevorzugt ist das Alkyl linear und bevorzugt ist das Alkyl Methyl, Ethyl, n-Propyl und n-Butyl.

R₃ enthält als Cycloalkyl bevorzugt 4 bis 7 und besonders bevorzugt 5 oder 6 Ringkohlenstoffatome. Beispiele für Cycloalkyl sind Cyclopropyl, Cyclobutyl, Cyclopentyl, Cyclohexyl, Cycloheptyl und Cyclooctyl. Besonders bevorzugt ist Cyclohexyl.

R₃ enthält als Cycloalkyl-alkyl bevorzugt 4 bis 7 und besonders bevorzugt 5 oder 6 Ringkohlenstoffatome, und bevorzugt 1 bis 4 und besonders bevorzugt 1 oder 2 C-Atome im aliphatischen Rest. Beispiele für Cycloalkyl-alkyl sind Cyclopropylmethyl oder -ethyl, Cyclobutylmethyl oder -propyl, Cyclopentylmethyl oder -ethyl, Cyclohexylmethyl oder -ethyl, Cycloheptylmethyl und Cyclooctylmethyl. Besonders bevorzugt ist Cyclohexylmethyl oder -ethyl.

R₃ kann als Aryl für Naphthyl und bevorzugt Phenyl stehen. R₂ ist als Aralkyl bevorzugt Phenylalkyl mit bevorzugt 1 bis 4 C-Atomen im Alkyl. Beispiele sind Benzyl und β-Phenylethyl.

X₂ in Formel IV steht besonders bevorzugt für -S(O)₂-.

Einige bevorzugte Beispiele für organische Säuren sind Essig-, Propion-, Butter-, Mono-, Di- und Trichloressig-, Mono-, Di- und Trifluoressig-, Hydroxyessig-, Oxal-, Malon-, Cyclohexanmono- und -dicarbonsäure, Benzoesäure, Phthal- und Terephthalsäure, Trifluormethylbenzoesäure, Phenylessigsäure, Phenylphosphonsäure, Methyl-, Ethyl-, Propyl-, Butyl-, Cyclohexyl-, Phenyl-, Methylphenyl-, Trifluormethylphenyl-, Mono-, Di- und Trichlormethyl-, und Mono-, Di- und Trifluormethylsulfonsäure. Besonders bevorzugt sind unsubstituierte und substituierte Phenylsulfonsäuren.

Die (αS)- beziehungsweise (αR)-Enantiomeren der Folsäureester können der Formel IIIa entsprechen, 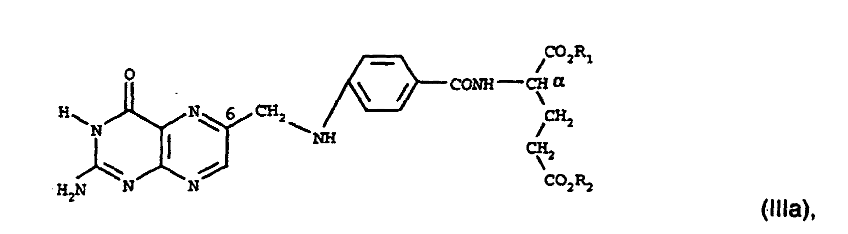 worin R₁ und R₂ die für die Verbindungen der Formel III angegebenen Bedeutungen haben, einschliesslich der Bevorzugungen.

Die Folsäure oder deren Carbonsäuresalze, Folsäureester und Folsäureestersalze und deren Enantiomere können teilweise oder vollständig im Reaktionsmedium gelöst sein. Bei teilweiser Lösung liegt eine Suspension oder Emulsion vor. Es hat sich als zweckmässig erwiesen, dass Folsäure oder deren Carbonsäuresalze, Folsäureester und Folsäureestersalze wenigstens zu 0,5 g pro Liter Lösungsmittel im Reaktionsmedium, bevorzugt wenigstens zu 1 g pro Liter, besonders bevorzugt wenigstens zu 5 g pro Liter und insbesondere bevorzugt wenigstens zu 10 g pro Liter gelöst sind.

Das Verfahren kann unter einem Wasserstoffdruck von 1 bis 500, bevorzugt 1 bis 150, besonders bevorzugt 1 bis 120, und insbesondere bevorzugt 5 bis 100 bar durchgeführt werden.

Die Reaktionstemperatur kann zum Beispiel 0 bis 150°C, bevorzugt 10 bis 120°C und besonders bevorzugt 10 bis 100°C betragen.

Die Katalysatormenge richtet sich hauptsächlich nach der gewünschten Reaktionszeit und nach wirtschaftlichen Überlegungen. Höhere Katalysatormengen begünstigen im wesentlichen kürzere Reaktionszeiten. Das molare Verhältnis von Substrat zu Katalysator kann zum Beispiel 10 bis 100000, bevorzugt 20 bis 20000, besonders bevorzugt, 50 bis 10000 und insbesondere 100 bis 5000 betragen.

Wässriges Reaktionsmedium bedeutet im Rahmen der Erfindung, dass nur Wasser oder Wasser in Abmischung mit einem organischen Lösungsmittel vorliegt. Der Anteil an Wasser beträgt bevorzugt wenigstens 30, besonders bevorzugt wenigstens 50 und insbesondere wenigstens 70 Volumenprozent. Ganz besonders bevorzugt enthält das Reaktionsmedium nur Wasser. Geeignete Lösungsmittel sind zum Beispiel Alkohole wie Methanol, Ethanol, Propanol, Butanol, Ethylenglykol, und Ethylenglykolmonomethylether; Ether wie Diethylether, Diisobutylether, Tetrahydrofuran und Dioxan; Sulfoxide und Sulfone wie Dimethylsulfoxid, Dimethylsulfon. Tetramethylensulfon; und N-substituierte Carbonsäureamide und Lactame wie N-Methylpyrrolidon und Dimethylformamid. Wenn Lösungsmittel nicht mit Wasser mischbar sind, erfolgt eine Zweiphasenhydrierung.

Dem wässrigen Reaktionsmedium können Puffermittel, Basen und/oder Säuren zugesetzt werden. Die Reaktion kann zum Beispiel bei einem pH-Wert von 1 bis 10, bevorzugt 3 bis 9 und besonders bevorzugt 5 bis 8 durchgeführt werden.

Geeignete Puffermittel sind insbesondere Phosphatpuffer; es können aber auch Carbonsäuren, Kohlensäure, Phosphorsäure und Borsäure verwendet werden. Geeignete Basen sind zum Beispiel Alkali- und Erdalkalimetallhydroxide, Amine, und Alkalimetallsalze von Carbonsäuren, Kohlensäure, Phosphorsäure und Borsäure. Geeignete Säuren sind zum Beispiel HCl, HBr, Hl, HBF₄, HClO₄, Carbonsäuren (gegebenenfalls fluorierte oder chlorierte Essigsäure, Benzoesäure, Zitronensäure), Borsäure, Phosphorsäure, Methansulfonsäure, Schwefelsäure und Kohlensäure. Bei den Basen und Säuren kann es sich auch um lösliche oder unlösliche Polymere wie zum Beispiel Ionenaustauscher handeln. Die Menge an Basen, Säuren und/oder Puffermitteln kann zum Beispiel 0 bis 2, bevorzugt 0 bis 1, und besonders bevorzugt 0 bis 0,5 Mol pro Liter Wasser betragen.

Alkoholisches Reaktionsmedium bedeutet im Rahmen der Erfindung die Gegenwart eines Alkohols, alleine oder in Abmischung mit einem anderen organischen Lösungsmittel. Geeignete Alkohole sind aliphatische, cycloaliphatische, cycloaliphatisch-aliphatische und araliphatische Alkohole. Einige bevorzugte Beispiele sind Methanol, Ethanol, n- oder i-Propanol, n-, i- oder t-Butanol, Pentanol, Hexanol, Cyclohexanol, Cyclohexandiol, Hydroxymethyl- oder Dihydroxymethylcyclohexan, Benzylalkohol, Ethylenglykol, Diethylenglykol, Propandiol, Butandiol, Ethylenglykolmonomethyl- oder -monoethylether, und Diethylenglykolmonomethyl- oder -monoethylether. Bevorzugt sind Methanol, Ethanol, Ethylenglykol, 1,2-Propandiol und i-Propanol. Der Anteil an einem Alkohol beträgt bevorzugt wenigstens 30, besonders bevorzugt wenigstens 50 und insbesondere wenigstens 70 Volumenprozent. Ganz besonders bevorzugt wird nur ein Alkohol verwendet. Die Stereoselektivität der Hydrierung hängt auch vom verwendeten Reaktionsmedium ab. Wenn ein Lösungsmittel nicht mit Alkohol mischbar ist, erfolgt eine Zweiphasenhydrierung.

Geeignete organische Lösungsmittel sind zum Beispiel Ether wie Diethylether, Diisobutylether, Tetrahydrofuran und Dioxan; Sulfoxide und Sulfone wie Dimethylsulfoxid, Dimethylsulfon, Tetramethylensulfon; N-substituierte Carbonsäureamide und Lactame wie N-Methylpyrrolidon und Dimethylformamid; Ketone wie Aceton oder Methyl-isobutylketon; und Carbonsäureester wie Essigsäuremethylester, Essigsäureethylester, Propionsäuremethylester.

Bevorzugte Katalysatormetalle sind Rhodium, Iridium und Ruthenium. Unter Katalysatoren werden auch Katalysatorvorläufer verstanden, die vor oder bei der Hydrierung durch den Kontakt mit Wasserstoff in katalytisch aktive Spezies umgewandelt werden.

Es ist bekannt, dass die katalytischen Eigenschaften der verwendeten Diphosphinkatalysatoren durch die Zugabe von Metallhalogeniden und Ammoniumhalogeniden beeinflusst werden können. Es kann daher vorteilhaft sein, dem Reaktionsgemisch Alkalimetall- oder Ammoniumchloride, -bromide oder -iodide zuzugeben, zum Beispiel LiCl, LiBr, Lil, Nal, NaBr oder Tetrabutylammoniumiodid. Die Menge kann zum Beispiel 0,001 bis 5 Mol pro Liter Lösungsmittel betragen. Femer können andere Modifikatoren und Co-Katalysatoren zugegen sein, zum Beispiel Phthalimide, Hydantoin oder Parabansäure.

Geeignete Liganden für Metallkomplexe sind zum Beispiel tertiäre Phosphine, besonders Triarylphosphine, zum Beispiel Triphenyl-, Tritoluyl- und Trixylylphosphin, und Tricycloalkylphosphine, zum Beispiel Tricyclohexylphosphin, sowie tertiäre Phosphane, zum Beispiel Tetramethylen- oder Pentamethylen-phenylphosphin. Besonders geeignet sind bidentate Liganden, wie zum Beispiel achirale oder chirale ditertiäre Diphosphine, oder Tertiärphosphinoimine. Beispiele für Letztere sind 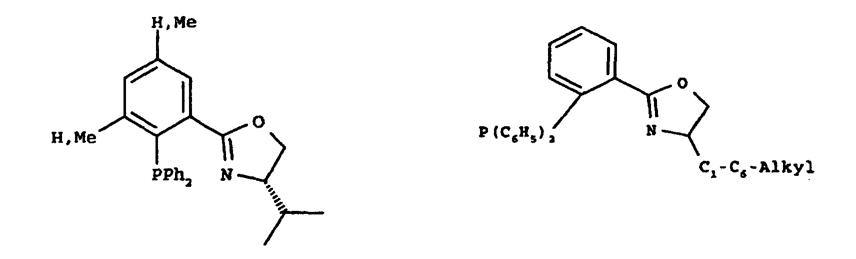 die von A. Lightfoot et al. in Angew. Chem. Int. Ed. 1998, 37, No. 20, Seiten 2897-2899 und P. Schnider et al., Chem. Eur. J., 1997, Bd.3, No. 6 beschrieben sind.

Achirale ditertiäre Diphosphine und chirale ditertiäre Diphosphine für asymmetrische Hydrierkatalysatoren in einem alkoholischen Reaktionsmedium sind in grosser Zahl in der Literatur beschrieben, siehe zum Beispiel H. Brunner und W. Zettimeier, Handbook of Enantioselective Catalysis, Vol. II: Ligand References, VCH Verlagsgesellschaft mbH Weinheim (1993).

Bei den achiralen und chiralen ditertiären Diphosphinen kann es sich um solche handeln, bei denen die Phosphingruppen (a) an verschiedene C-Atome einer Kohlenstoffkette mit 2 bis 4 C-Atomen, oder (b) direkt oder über eine Brückengruppe -CRₐR_{b}- in den Orthostellungen eines Cyclopentadienylrings oder an je einen Cyclopentadienyl eines Ferrocenyls gebunden sind, wobei Rₐ und R_{b} gleich oder verschieden sind und für H, C₁-C₈-Alkyl, C₁-C₄-Fluoralkyl, C₅-C₆-Cycloalkyl, Phenyl, Benzyl, oder mit 1 bis 3 C₁-C₄-Alkyl oder C₁-C₄-Alkoxy substituiertes Phenyl oder Benzyl stehen. R_{b} steht bevorzugt für Wasserstoff. Rₐ bedeutet bevorzugt C₁-C₄-Alkyl.

Die Phosphingruppen enthalten bevorzugt zwei gleiche oder verschiedene, bevorzugter gleiche unsubstituierte oder substituierte Kohlenwasserstoffreste mit 1 bis 20, bevorzugt 1 bis 12 C-Atomen. Unter den ditertiären Diphosphinen sind solche besonders bevorzugt, worin die beiden Phosphingruppen zwei gleiche oder verschiedene Reste ausgewählt aus der Gruppe lineares oder verzweigtes C₁-C₁₂-Alkyl; unsubstituiertes oder mit C₁-C₆-Alkyl oder C₁-C₆-Alkoxy substituiertes C₅-C₁₂-Cycloalkyl, C₅-C₁₂-Cycloalkyl-CH₂-, Phenyl oder Benzyl; oder mit Halogen (zum Beispiel F, Cl und Br), C₁-C₆-Alkyl, C₁-C₆-Halogenalkyl (zum Beispiel Trifluormethyl), C₁-C₆-Alkoxy, C₁-C₆-Halogenalkoxy (zum Beispiel Trifluormethoxy), (C₆H₅)₃Si, (C₁-C₁₂-Alkyl)₃Si, -NH₂, -NH(C₁-C₁₂-Alkyl), -NH(Phenyl), -NH(Benzyl), -N(C₁-C₁₂-Alkyl)₂, -N (Phenyl)₂, -N(Benzyl)₂, Morpholinyl, Piperidinyl, Pyrrolidinyl, Piperazinyl, -Ammonium-X₃-, -SO₃M₁, -CO₂M₁, -PO₃M₁, oder -CO₂-C₁-C₆-Alkyl (zum Beispiel -CO₂CH₃) substituiertes Phenyl oder Benzyl enthalten, worin M, ein Alkalimetall oder Wasserstoff darstellt, und X₃⁻ das Anion einer einbasischen Säure ist. M₁ steht bevorzugt für H, Li, Na und K. X₃⁻ stellt als Anion einer einbasischen Säure bevorzugt Cl⁻, Br⁻, oder das Anion einer Monocarbonsäure dar, zum Beispiel Formiat, Acetat, Trichloracetat oder Trifluoracetat.

Die beiden Reste der Phosphingruppen können je zusammen auch unsubstituiertes oder mit Halogen, C₁-C₆-Alkyl oder C₁-C₆-Alkoxy substituiertes Tetramethylen, Pentamethylen oder 3-Oxa-pentan-1,5-diyl bedeuten. Die Substituenten sind bevorzugt in den Orthostellen zum P-Atom gebunden.

Bei den Phosphingruppen kann es sich auch um solche der Formeln 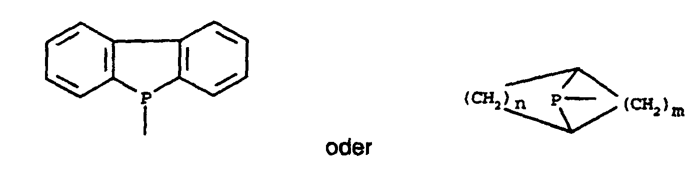 handeln, worin m und n unabhängig voneinander eine ganze Zahl von 2 bis 10 sind, und die Summe von m+n 4 bis 12 und bevorzugt 5 bis 8 ist. Beispiele sind [3.3.1]- und [4.2.1]-Phobyl der Formeln

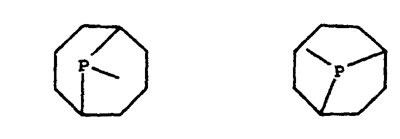

Beispiele für Alkyl, das bevorzugt 1 bis 6 C-Atome enthält, sind Methyl, Ethyl, n-Propyl, i-Propyl, n-Butyl, i-Butyl, t-Butyl, und die Isomeren von Pentyl und Hexyl. Beispiele für gegebenenfalls mit Alkyl substituiertes Cycloalkyl sind Cyclopentyl, Cyclohexyl, Methyl- und Ethylcyclohexyl, und Dimethylcyclohexyl. Beispiele für mit Alkyl, Alkoxy, Halogenalkyl und Halogenalkoxy substituiertes Phenyl und Benzyl sind Methylphenyl, Dimethylphenyl, Trimethylphenyl, Ethylphenyl, Methylbenzyl, Methoxyphenyl, Dimethoxyphenyl, Trifluormethylphenyl, Bis-trifluormethylphenyl, Tris-trifluormethylphenyl, Trifluormethoxyphenyl und Bis-trifluormethoxyphenyl.

Bevorzugte Phosphingruppen sind solche, die gleiche oder verschiedene und bevorzugt gleiche Reste ausgewählt aus der Gruppe C₁-C₆-Alkyl, unsubstituiertes oder mit 1 bis 3 C₁-C₄-Alkyl oder C₁-C₄-Alkoxy substituiertes Cyclopentyl oder Cyclohexyl, Benzyl und besonders Phenyl, das unsubstituiert oder substituiert ist mit 1 bis 3 C₁-C₄-Alkyl, C₁-C₄-Alkoxy, F, Cl, C₁-C₄-Fluoralkyl oder C₁-C₄-Fluoralkoxy, enthalten.

Die Diphosphine entsprechen bevorzugt der Formel IV,

R₄R₅P-R₆-PR₇R₈ (IV),

worin
R₄, R₅, R₇ und R₈ unabhängig voneinander einen Kohlenwasserstoffrest mit 1 bis 20 C-Atomen darstellen, die unsubstituiert oder substituiert sind mit Halogen, C₁-C₆-Alkyl, C₁-C₆-Halogenalkyl, C₁-C₆-Alkoxy, C₁-C₆-Halogenalkoxy, (C₆H₅)₃Si, (C₁-C₁₂-Alkyl)₃Si, -NH₂, -NH(C₁-C₁₂-Alkyl), -NH(Phenyl), -NH(Benzyl), -N(C₁-C₁₂-Alkyl)₂, -N (Phenyl)₂, -N(Benzyl)₂, Morpholinyl, Piperidinyl, Pyrrolidinyl, Piperazinyl, -Ammonium-X₃-, -SO₃M₁, -CO₂M₁, -PO₃M₁, oder -CO₂-C₁-C₆-Alkyl, worin M₁ ein Alkalimetall oder Wasserstoff darstellt, und X₃⁻ das Anion einer einbasischen Säure ist; oder R₄ und R₅ sowie R₇ und _{R8} je zusammen unsubstiuiertes oder mit Halogen, C₁-C₆-Alkyl oder C₁-C₆-Alkoxy substituiertes Tetramethylen, Pentamethylen oder 3-Oxa-pentan-1,5-diyl bedeuten; und
R₆ unsubstituiertes oder mit C₁-C₆-Alkyl, C₁-C₆-Alkoxy, C₅- oder C₆-Cycloalkyl, Phenyl, Naphthyl oder Benzyl substituiertes C₂-C₄-Alkylen; unsubstituiertes oder mit C₁-C₆-Alkyl, Phenyl oder Benzyl substituiertes 1,2- oder 1,3-Cycloalkylen, 1,2-oder 1,3-Cycloalkenylen, 1,2- oder 1,3-Bicycloalkylen oder 1,2- oder 1,3-Bicycloalkenylen mit 4 bis 10 C-Atomen; unsubstituiertes oder mit C₁-C₆-Alkyl, Phenyl oder Benzyl substituiertes 1,2- oder 1,3-Cycloalkylen, 1,2- oder 1,3-Cycloalkenylen, 1,2- oder 1,3-Bicycloalkylen oder 1,2- oder 1,3-Bicycloalkenylen mit 4 bis 10 C-Atomen, an deren 1- und/oder 2-Stellungen oder an deren 3-Stellung Methylen oder C₂-C₄-Alkyliden gebunden ist; in den 2,3-Stellungen mit R₉R₁₀C(O-)₂ substituiertes 1,4-Butylen, das in den 1- und/oder 4-Stellungen unsubstituiert oder mit C₁-C₆-Alkyl, Phenyl oder Benzyl substituiert ist, und wobei R₉ und R₁₀ unabhängig voneinander Wasserstoff, C₁-C₆-Alkyl, Phenyl oder Benzyl darstellt; 3,4- oder 2,4-Pyrrolidinylen oder Methylen-4-pyrrolidin-4-yl, deren N-Atom substituiert ist mit Wasserstoff, C₁-C₁₂-Alkyl, Phenyl, Benzyl, C₁-C₁₂-Alkoxycarbonyl, C₁-C₈-Acyl, C₁-C₁₂-Alkylaminocarbonyl; oder unsubstituiertes oder mit Halogen, -OH, C₁-C₆-Alkyl, C₁-C₆-Alkoxy, Phenyl, Benzyl, Phenyloxy oder Benzyloxy substituiertes 1,2-Phenylen, 2-Benzylen, 1,2-Xylylen, 1,8-Naphthylen, 2,2'-Dinaphthylen oder 2,2'-Diphenylen bedeutet; oder R₆ für einen Rest der Formeln 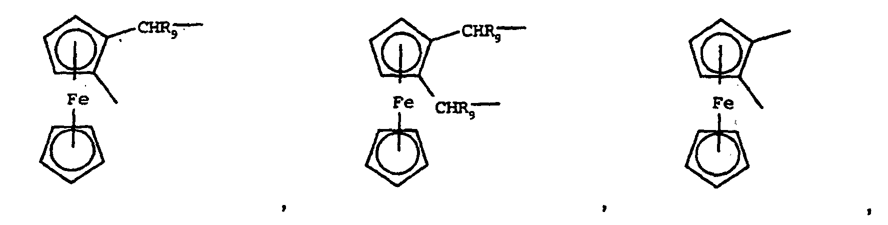 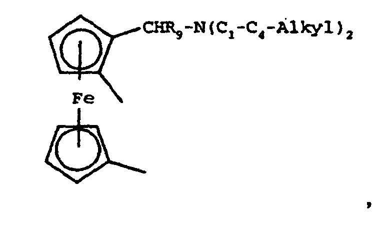 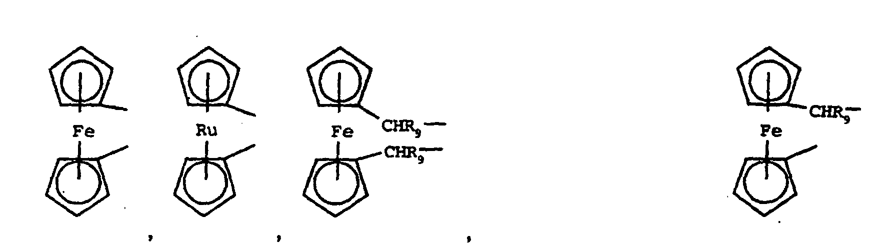 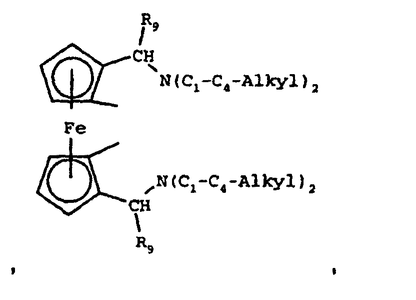 steht, worin R₉ Wasserstoff, C₁-C₈-Alkyl, C₁-C₄-Fluoralkyl, unsubstituiertes oder mit 1 bis 3 F, Cl, Br, C₁-C₄-Alkyl, C₁-C₄-Alkoxy oder Fluormethyl substituiertes Phenyl bedeutet.

Bevorzugt sind R₄, R₅, R₇ und R₈ gleiche oder verschiedene und insbesondere gleiche Reste, ausgewählt aus der Gruppe C₁-C₆-Alkyl, unsubstituiertes oder mit ein bis drei C₁-C₄-Alkyl oder C₁-C₄-Alkoxy substituiertes Cyclopentyl oder Cyclohexyl, unsubstituiertes oder mit ein bis drei C₁-_{C4}-Alkyl oder C₁-C₄-Alkoxy substituiertes Benzyl, und insbesondere unsubstituiertes oder mit ein bis drei C₁-C₄-Alkyl, C₁-C₄-Alkoxy, -NH₂, OH, F, Cl, C₁-C₄-Fluoralkyl oder C₁-C₄-Fluoralkoxy substituiertes Phenyl.

Eine bevorzugte Gruppe an achiralen und chiralen Diphosphinen sind solche der Formeln V bis XXIII, 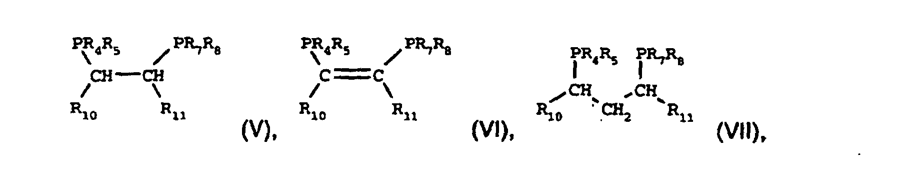 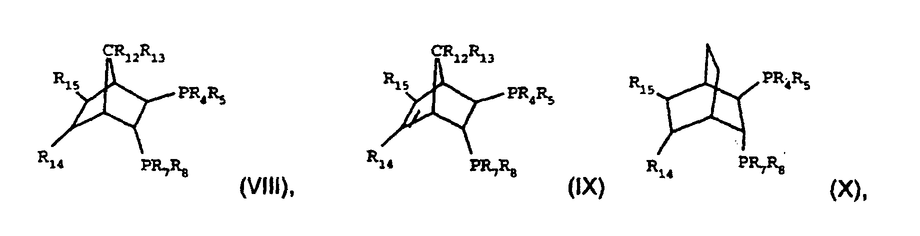 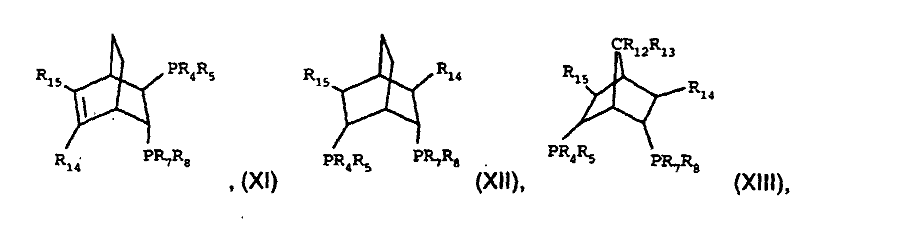 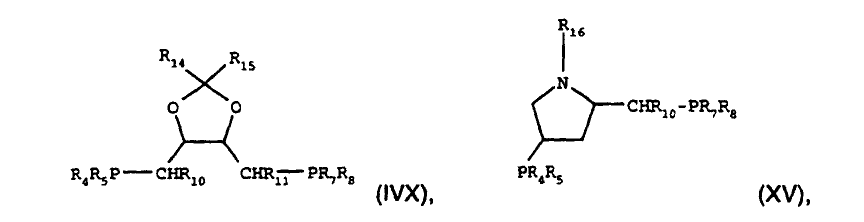 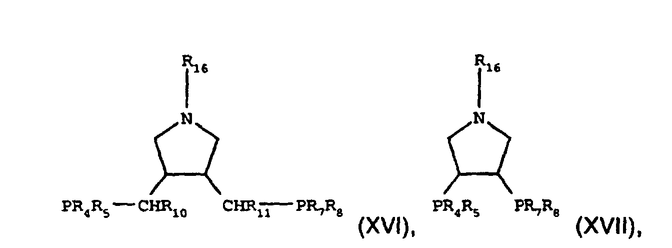 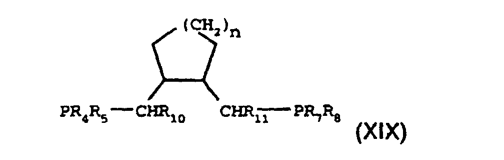 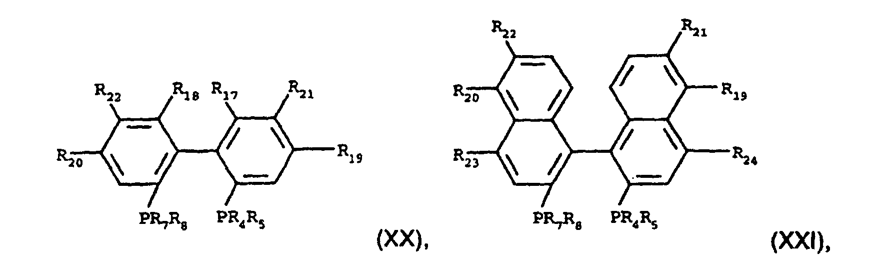 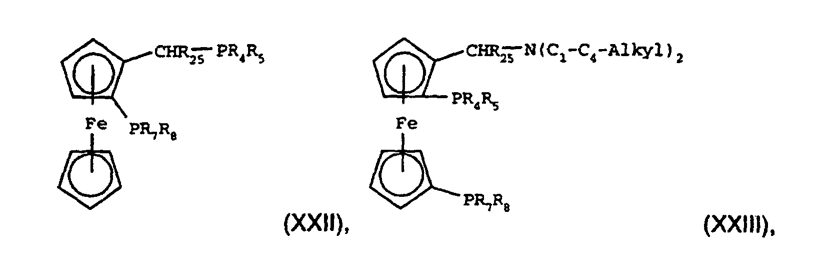 worin R₄, R₅ R₇ und R₈ die zuvor angegebenen Bedeutungen haben, einschliesslich der Bevorzugungen,
R₁₀ und R₁₁ unabhängig voneinander Wasserstoff, C₁-C₄-Alkyl oder unsubstituiertes oder mit ein bis drei C₁-C₄-Alkyl oder C₁-C₄-Alkoxy substituiertes Benzyl oder Phenyl bedeuten,
R₁₂ und R₁₃ unabhängig voneinander Wasserstoff, C₁-C₄-Alkyl, Phenyl oder Benzyl darstellen,
R₁₄ und R₁₅ unabhängig voneinander Wasserstoff, C₁-C₄-Alkyl, C₁-C₄-Alkoxy oder unsubstituiertes oder mit ein bis drei C₁-C₄-Alkyl oder C₁-C₄-Alkoxy substituiertes Benzyl oder Phenyl bedeuten,
R₁₆ Wasserstoff, C₁-C₁₂-Alkyl, unsubstituiertes oder mit ein bis drei C₁-C₄-Alkyl oder C₁-C₄-Alkoxy substituiertes Benzyl oder Phenyl, C₁-C₁₂-Alkoxy-C(O)-, unsubstituiertes oder mit ein bis drei C₁-C₄-Alkyl oder C₁-C₄-Alkoxy substituiertes Phenyl-C(O)- oder Benzyl-C(O)-, C₁-C₁₂-Alkyl-NH-CO-, oder unsubstituiertes oder mit ein bis drei C₁-C₄-Alkyl oder C₁-C₄-Alkoxy substituiertes Phenyl-NH-C(O)- oder Benzyl-NH-C(O)- darstellt,
n für 0, 1 oder 2 steht,
R₁₇ und R₁₈ C₁-C₄-Alkyl oder C₁-C₄-Alkoxy sind, oder R₁₇ und R₁₈ zusammen Oxadimethylen bedeuten,
R₁₉, R₂₀, R₂₁, R₂₂, R₂₃ und R₂₄ und unabhängig voneinander H, C₁-C₄-Alkyl, C₁-C₄-Alkoxy, C₅- oder C₆-Cycloalkyl oder -alkoxy, Phenyl, Benzyl, Phenoxy, Benzyloxy, Halogen, OH, -(CH₂)₃-C(O)-O-C₁-C₄-Alkyl, -(CH₂)₃-C(O)-N(C₁-C₄-Alkyl)₂ oder -N(C₁-C₄-Alkyl)₂ sind, oder R₁₉ und R₂₁, und/oder R₁₇ und R₂₁, und/oder R₂₀ und R₂₂, und/oder R₁₈ und R₂₂, oder R₂₁ und R₂₃ und/oder R₂₂ und R₂₄ je zusammen einen ankondensierten 5- oder 6-gliedrigen, mono- oder bicyclischen Kohlenwasserstoffring darstellen, und
R₂₅ C₁-C₄-Alkyl ist.

Einige bevorzugte Beispiele chiraler ditertiärer Diphosphine sind solche der nachfolgenden Formeln V bis XL: 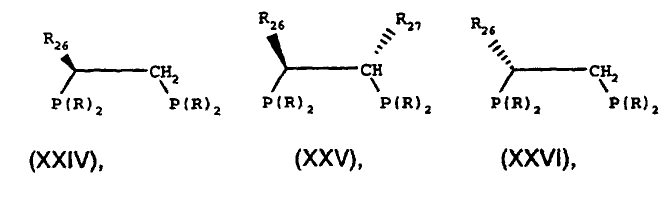 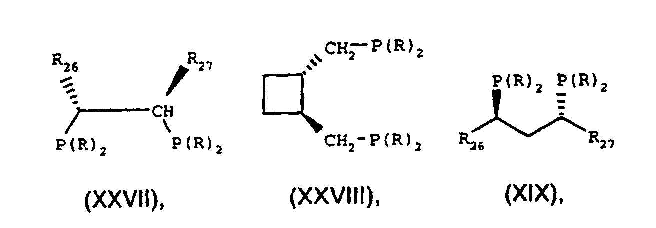 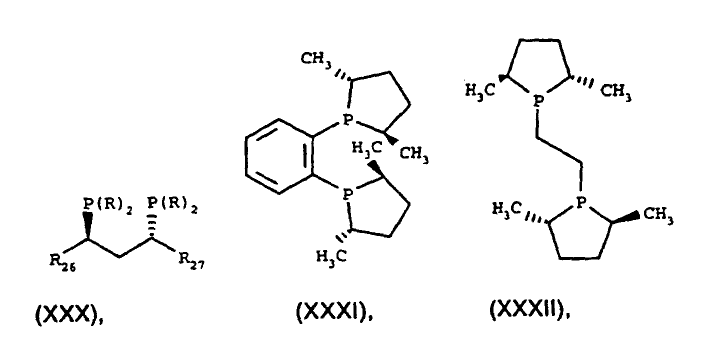 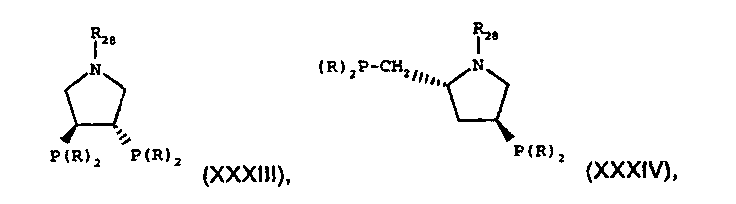 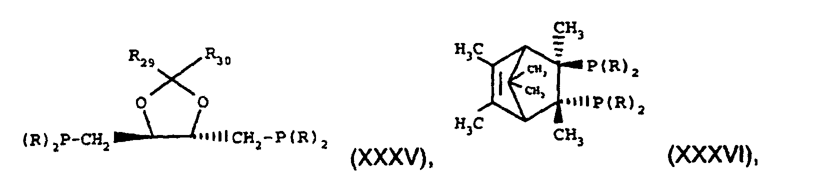 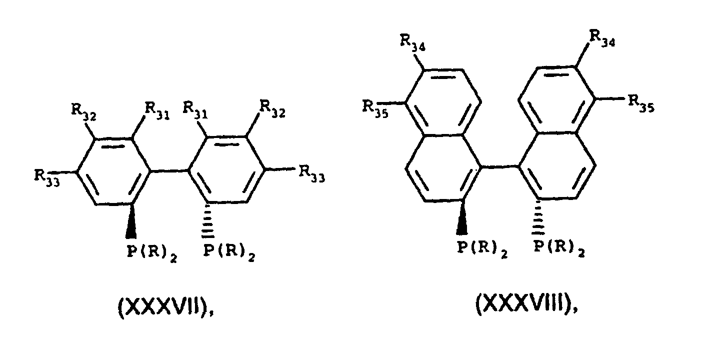 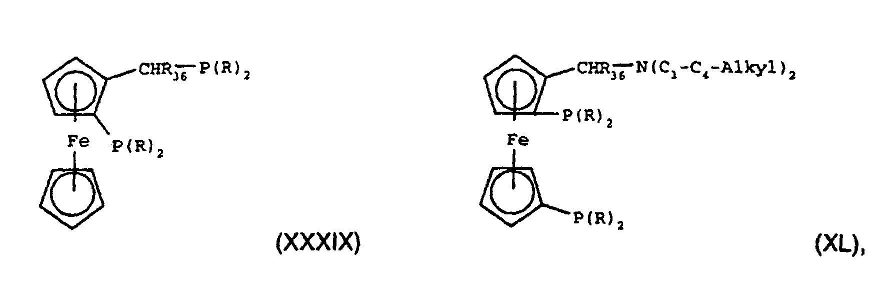 worin
R für Cyclohexyl oder unsubstituiertes oder mit ein bis drei C₁-C₄-Alkyl, C₁-C₄-Alkoxy, Trifluormethyl, oder ein -NH₂ (C₁-C₄-Alkyl)NH-, (C₁-C₄-Alkyl)₂N- substituiertes Phenyl steht,
R₂₆ und R₂₇ unabhängig voneinander C₁-C₄-Alkyl, Phenyl oder Benzyl und besonders bevorzugt Methyl bedeuten,
R₂₈ C₁-C₈-Alkyl, C₁-C₈-Acyl oder C₁-C₈-Alkoxycarbonyl darstellt,
R₂₉ für Wasserstoff steht oder unabhängig die Bedeutung von R₃₀ hat, und R₃₀ C₁-C₄-Alkyl, Phenyl oder Benzyl darstellt,
R₃₁ Methyl, Methoxy oder beide R₃₁ zusammen Oxadimethylen bedeuten,
R₃₂ und R₃₃ unabhängig voneinander H, C₁-C₄-Alkyl, C₁-C₄-Alkoxy oder (C₁-C₄-Alkyl)₂N- darstellen,
R₃₄ und R₃₅ unabhängig voneinander H, C₁-C₄-Alkyl, C₁-C₄-Alkoxy, -(CH₂)₃-C(O)-O-C₁-C₄-Alkyl, -(CH₂)₃-C(O)-N(C₁-C₄-Alkyl)₂ oder ein Paar R₃₄ und R₃₅ zusammen einen Rest der Formel XLI und das andere Paar R₃₄ und R₃₅ zusammen einen Rest der Formel XLII 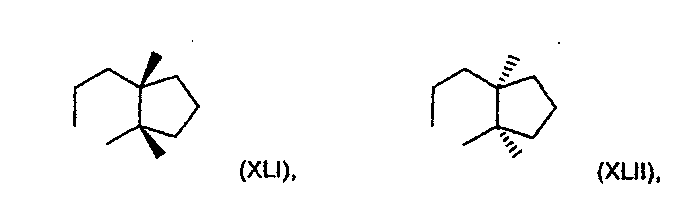 darstellen, und
R₃₆ für C₁-C₄-Alkyl und besonders bevorzugt Methyl steht.

Geeignete ditertiäre Diphosphine mit heterocyclischen Gerüsten sind beschrieben in EP 0 770 085, von T. Benincori et al. in J. of Organomet. Chem. 529 (1997), Seiten 445 bis 453 und in J. Org. Chem., 61, S. 6244, (1996) von F. Bonifacio et al. in Chiratech 1997, 11. bis 13. November 1997, Philadelphia, Pennsylvania, USA und von L.F. Tietze et al, Chem. Commun. S. 1811-1812 (1999)
Einige Beispiele sind 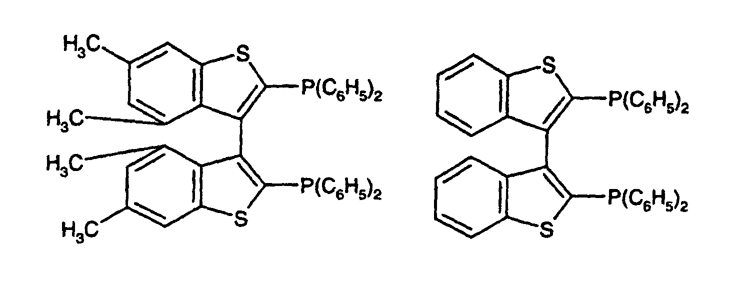 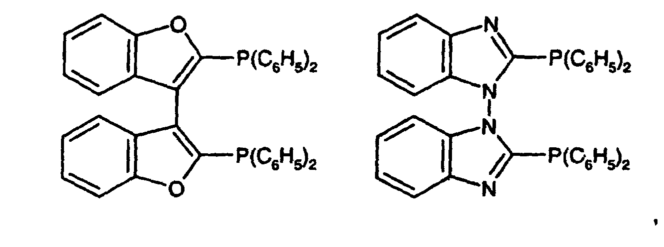 und

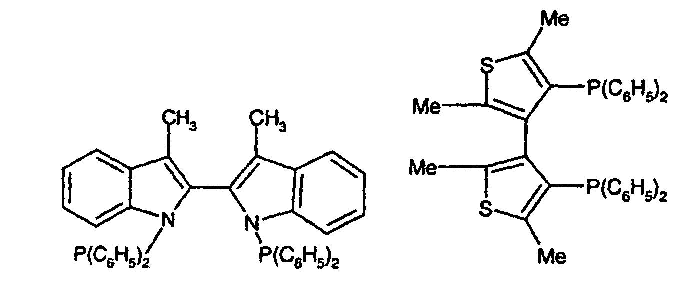

Achirale und chirale ditertiäre Diphosphine für wasserlösliche Katalysatoren sind ebenfalls bekannt und in der Literatur beschrieben. Solche Diphosphine enthalten eine oder mehrere wasser-solubilisierende polare Substituenten, die entweder direkt oder über eine Brückengruppe an Substituenten der Phosphingruppen und/oder an das Gerüst des Diphosphins gebunden sind. Bei den Diphosphinen kann es sich um die gleichen achiralen und chiralen ditertiären Diphosphine wie zuvor definiert handeln, einschliesslich der Bevorzugungen, die zusätzlich wasser-solubilisierende polare Substituenten enthalten. Solche Liganden sind zum Beispiel von G. Papadogianakis et al. in James J. Spivey (Editor), Catalysis Vol. 13, The Royal Society of Chemistry / Information Service (1997), Seiten 115-193 beschrieben.

Bei den polaren Substituenten kann es sich um Hydroxyl und Säure- oder Ammmoniumgruppen handeln. Beispiele für Säuregruppen sind Carbon-, Sulfon-, Sulfat- und Phosphonsäuregruppen. Beispiele für Ammonium sind -NH₃⁺ sowie sekundäres Ammonium mit 1 bis 12, vorzugsweise 1 bis 6 C-Atomen; tertiäres Ammonium mit 2 bis 24, vorzugsweise 2 bis 12 C-Atomen; und quarternäres Ammonium mit 3 bis 36, vorzugsweise 3 bis 18 C-Atomen, wobei die Ammoniumgruppen ein Anion einer anorganischen oder organischen Säure enthalten.

Eine ganz besonders bevorzugte Gruppe polarer Substituenten ist ausgewählt aus der Gruppe -OH, -CO₂M₁, -SO₃M₁, -O-SO₃M₁, -PO(OM₁)2, und -NR₃₇R₃₈R₃₉⁺X₄⁻, worin M, für H, ein Alkalimetallkation oder ein Ammoniumkation steht, R₃₇, R₃₈ und R₃₉ unabhängig voneinander H, C₁-C₄-Alkyl, Phenyl oder Benzyl, oder R₃₇ und R₃₈ zusammen Tetramethylen, Pentamethylen oder 3-Oxapentylen sind, und X₄ das Anion einer anorganischen oder organischen Säure ist. Beispiele für Säuren, von denen sich das Anion ableiten kann, sind HCl, HBr, Hl, H₂SO₄, C₁-C₈-Carbonsäuren, C₁-C₈-Sulfonsäuren, C₁-C₈-Phosphonsäuren, HClO₄, HBF₄, HSbF₆ und HPF₆. M₁ kann als Ammoniumkation der Formel ⁺NR₃₇R₃₈R₃₉R₄₀ entsprechen, R₃₇, R₃₈, R₃₉ und R₄₀ unabhängig voneinander H, C₁-C₄-Alkyl, Phenyl oder Benzyl, oder R₃₇ und R₃₈ zusammen Tetramethylen, Pentamethylen oder 3-Oxapentylen sind. Die Phosphingruppen können 1 bis 4 polare Substituenten enthalten, wobei wenigstens ein Rest der Phosphingruppe wenigstens einen polaren Substituenten enthält.

Erfindungsgemäss werden auch ditertiäre Diphosphine umfasst, deren Wasserlöslichkeit durch eine (direkte oder über eine Brückengruppe) kovalente Bindung des Diphosphins an das Rückgrat eines wasserlöslichen Polymers oder Oligomers erzielt wird, zum Beispiel Polyethylenglykol, Polyvinylalkohol und Polyacrylsäure.

Bei den Brückengruppen kann es sich um solche der Formel -X₅-R₄₁- handeln, worin X₅ eine direkte Bindung, O, NH, Si(CH₃)₂ N(C₁-C₄-Alkyl), NH-CO, N(C₁-C₄-Alkyl)CO, CO-NH, CON(C₁-C₄-Alkyl), NH-CO-O, N(C₁-C₄-Alkyl)CO-O, O-CO-NH, O-CON(C₁-C₄-Alkyl), NH-CO-NH, N(C₁-C₄-Alkyl)CO-NH oder N(C₁-C₄-Alkyl)CO-N(C₁-C₄-Alkyl) darstellt, und R₄₁ für einen mono- bis tetravalenten Kohlenwasserstoffrest mit 1 bis 40, vorzugsweise 1 bis 30, und besonders bevorzugt 1 bis 20 C-Atomen steht, der mit Heteroatomen oder Heterogruppen wie zuvor für X₅ genannt ein- oder mehrfach unterbrochen sein kann. Beispiele für Kohlenwasserstoffreste sind lineares oder verzweigtes C₁-C₁₈-Alkylen, C₅- oder C₆-Cycloalkylen, C₅-oder C₆-Cycloalkylen-C₁-C₆-Alkylen, C₅- oder C₁-C₆-Alkylen-C₆-Cycloalkylen-C₁-C₆-Alkylen, Phenylen, Phentriyl, C₁-C₆-Alkylen-C₆H₄-, C₁-C₆-Alkylen-C₆H₄-C₁-C₆-Alkylen, und (C₁-C₆-Alkylen)₃-C₆H₃-.

Eine bevorzugte Gruppe an achiralen und chiralen Diphosphinen sind solche der Formeln V bis XXIII und besonders bevorzugt Diphosphine der Formeln XXIV bis XL, worin
R₁₀ bis R₃₆ die zuvor angegebenen Bedeutungen haben,
R₄, R₅, R₇ und R₈ gleich sind und wie die beiden R für einen Rest der Formel 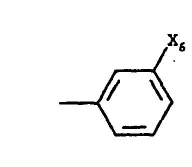 stehen,
worin X₆ -SO₃M₁, -CO₂M₁, -C₁-C₄-Alkylen-SO₃M₁, -C₁-C₄-Alkylen-CO₂M₁, - N(C₁-C₄-Alkyl)₂ oder ⁺N(C₁-C₄-Alkyl)₂X₄⁻ darstellt, M₁ H, Na oder K bedeutet, und X₄ für Cl, Br oder I steht.

Einige Beispiele für polymere wasserlösliche Diphosphine sind in den EP-0 329 043 und WO 98/01457 sowie von W.D. Müller et al. in Chem. Commun., (1996) Seiten 1135-1136 beschrieben.

Eine weitere bevorzugte Gruppe wasserlöslicher Diphosphine sind solche der Formel XLIII,

(M₁O₂C-CH₂CH₂-O-CH₂)₃C-NR₄₂-CO―R₄₁ (XLIII)

worin M₁ für H, ein Alkalimetallkation oder ein Ammoniumkation steht, R₄₂ C₁-C₄-Alkyl und vorzugsweise H bedeutet, und R₄₁ der monovalente Rest eines chiralen ditertiären Diphosphins ist, wobei die CO-Gruppe direkt an ein C- oder N-Atom des Diphosphingerüsts, oder an ein O- oder N-Atom oder an ein C-Atom einer Brückengruppe des Diphosphingerüsts gebunden ist. Geeignete Brückengruppen sind zum Beispiel -O-, -NH-, C₁-C₆-Alkylen-,
-N(C₁-C₄-Alkyl)-, -O-C₁-C₆-Alkylen-, -NH-C₁-C₆-Alkylen- und -N(C₁-C₄-Alkyl)-C₁-C₆-Alkylen-. Für M₁ gelten die zuvor angegebenen Ausgestaltungen und Bevorzugungen.

Eine bevorzugte Untergruppe der Diphosphine der Formel XLIII sind solche der Formel XLIIIa

(M₁O₂C-CH₂CH₂-O-CH₂)₃C-NH-CO-R₄₃ (XLIIIa)

worin M₁ die zuvor angegebenen Bedeutungen hat, und R₄₃ einen Rest der Formeln 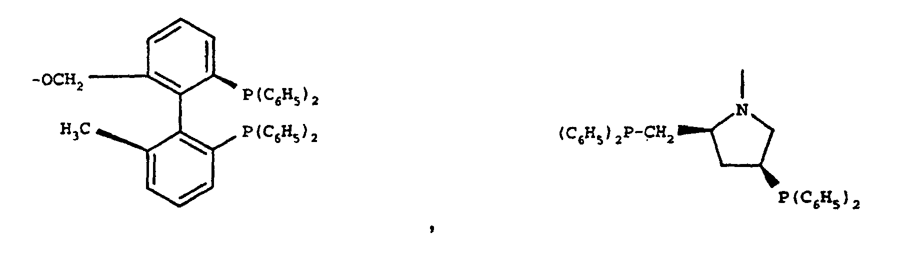 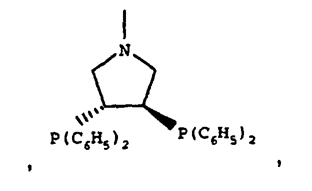 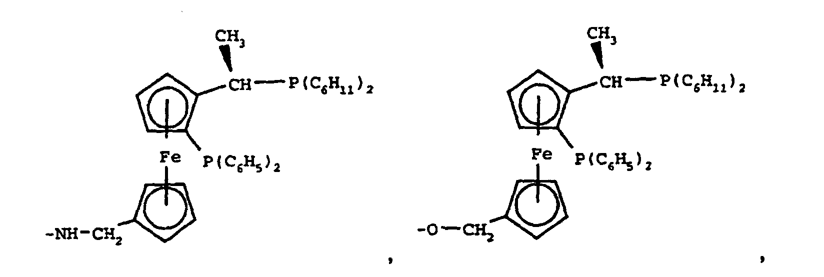 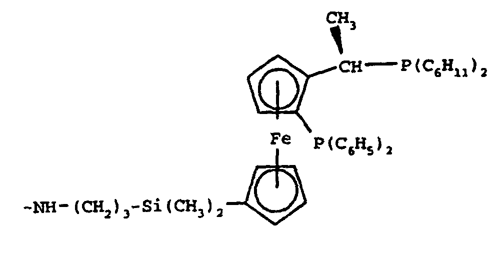 bedeutet.

Ein weiteres Beispiel für wasserlösliche Ferrocenyldiphosphine ist die Verbindung der Formel 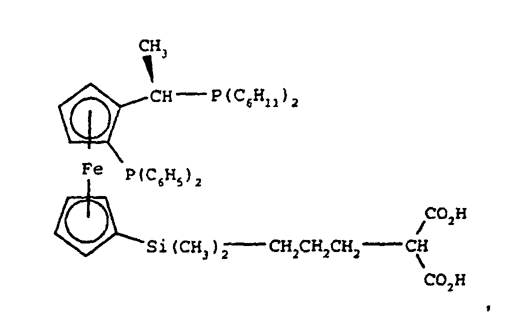 die in der WO 98/01457 beschrieben ist.

Ebenfalls miteingeschlossen sind Verbindungen der folgenden Form 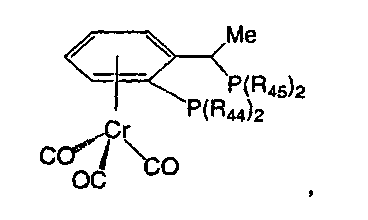 wobei R₄₄ und R₄₅ gleich oder verschieden sind und für Phenyl, o-Tolyl, p-Tolyl, m-Tolyl, Butyl, Propyl, Xylyl, Cyclohexyl, oder
Phoban 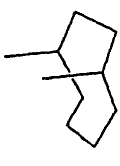 stehen
oder Verbindungen der Form 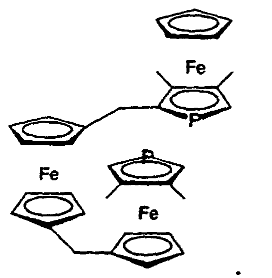 Entsprechende Verbindungen sind beschrieben in U. Englert et al. in Organo metallics (eingereicht) und A. Salzer et al. in Organometallics (eingereicht).

Die Diphosphine der Formeln XLIII und XLIIIa sind neu und auf folgende Weise erhältlich. Das bekannte Amin (HO₂C-CH₂CH₂-O-CH₂)₃C-NH₂ beziehungsweise seine Alkylester kann mit Carboxylgruppen eines entsprechenden ditertiären Diphosphins zum Amid umgesetzt werden. Das Amin kann zum Isocyanat oder einem verkappten Isocyanat (zum Beispiel mit Carbonyldiimidazol) derivatisiert werden, die mit OH- oder NH-Gruppen eines entsprechenden ditertiären Diphosphins unter Bildung von Urethan- oder Harnstoffbrücken umgesetzt werden können.

Bei den erfindungsgemäss verwendeten Katalysatoren beziehungsweise Katalysatorvorläufern kann es sich um Metallkomplexe der Formel XUV, XLIVa und XLIVb handeln,

[X₇Me₂YZ] (XLIV),

[X₇Me₂Y]⁺A₂⁻ (XLIVa),

[X₇Ru(II)X₈X₉] (XLIVb),

worin
Y für zwei Monoolefinliganden oder einen Dienliganden steht;
X₇ ein achirales oder chirales ditertiäres Diphosphin darstellt, das mit dem Metallatom Me₂ oder Ru einen 5- bis 7-gliedrigen Ring bildet;
Me₂ Ir(I) oder Rh(I) bedeutet;
Z -Cl, -Br oder -I darstellt; und
A₂ das Anion einer Sauerstoffsäure oder Komplexsäure ist,
X₈ und X₉ gleich oder verschieden sind und die Bedeutung von Z und A₂ haben, oder X₈ und ₓ₉ für Allyl oder 2-Methylallyl stehen, oder X₈ die Bedeutung von Z oder A hat und X₉ für Hydrid steht.

Bevorzugt sind Metallkomplexe, bei denen Y für 1,5-Hexadien, 1,5-Cyclooctadien oder Norbornadien steht. Bevorzugt steht in den erfindungsgemässen Metallkomplexen Z für -Cl, -Br oder -I. In bevorzugten Metallkomplexen steht A₂ für ClO₄-, CF₃SO₃⁻, CH₃SO₃⁻, HSO₄⁻, BF₄⁻, B(Phenyl)₄⁻, PF₆-, SbCl₆⁻, AsF₆⁻ oder SbF₆⁻.

Weitere in Frage kommende Rutheniumkomplexe sind literaturbekannt und zum Beispiel beschrieben in US 4,691,037, US 4,739,085, US 4,739,084, EP 0 269 395, EP 0 271 310, EP 0 271 311, EP 0 307 168, EP 0 366 390, EP 0 470 756, JP 08 081 484, JP 08 081 485, JP 09 294 932, EP 0 831 099, EP 0 826 694, EP 0 841 343, J. P. Genêt, Arcos Organics Acta, 1 (1995) 4, N.C. Zanetti et al., Organometallics 15 (1996) 860.

Die Metallkomplexe der Formeln XLIV, XLIVa oder XLIVb werden nach in der Literatur bekannten Methoden hergestellt. Die Herstellung ist zum Beispiel in der EP-0 564 406 beschrieben. Die Katalysatoren beziehungsweise Katalysatorvorläufer können als isolierte Verbindungen zum Reaktionsgemisch gegeben werden. Es hat sich als vorteilhaft erwiesen, die Katalysatoren beziehungsweise Katalysatorvorläufer vor der Umsetzung mit oder ohne Lösungsmittel in situ herzustellen und dann zur Umsetzung mit dem Reaktionsgemisch zu vereinigen.

Im Einzelnen kann das Verfahren so durchgeführt werden, dass man zunächst den Katalysator herstellt und dann den Katalysator zu einer Lösung oder Suspension der zu hydrierenden Pterine, zum Beispiel Folsäure oder deren Carbonsäuresalzen, Folsäureestern oder Folsäureestersalzen gibt oder umgekehrt. In einem Autoklaven presst man Wasserstoff auf und entfemt auf diese Weise ein zweckmässig verwendetes Schutzgas. Man erwärmt gegebenenfalls und hydriert dann das Reaktionsgemisch. Nach Beendigung der Reaktion wird gegebenenfalls abgekühlt und der Autoklav entspannt. Man kann das Reaktionsgemisch mit zum Beispiel Stickstoff aus dem Reaktor pressen und das hydrierte Reaktionsprodukt in an sich bekannter Weise isolieren, zum Beispiel mittels Extraktion, Fällung und Kristallisation, oder in situ weiter umsetzen. Es wurde beobachtet, dass (6S,αS)- und (6S,αR)-Tetrahydrofolsäureester und (6S,αR)-Tetrahydrofolsäureestersalze schon während der Hydrierung auszufallen vermögen, was die Isolierung aus dem Reaktionsgemisch erheblich erleichtern kann.

Es ist besonders vorteilhaft, wenn man Folsäure einsetzt, und eine Veresterung und Hydrierung nacheinander in einem Reaktionsgefäss durchführt. Zweckmässig verwendet man für die Veresterung in Gegenwart einer Säure HA das gleiche Lösungsmittel wie bei der Hydrierung, insbesondere den Alkohol, zum Beispiel Methanol oder Ethanol, mit dem die Folsäure auch verestert wird.

In einer anderen vorteilhaften Verfahrensvariante erfolgt die Veresterung der Folsäure und die Hydrierung gleichzeitig, wobei der Tetrahydrofolsäureester und seine Salze in situ gebildet und gleichzeitig hydriert wird. Hierzu gibt man alle Komponenten (Folsäure, Alkohol, Lösungsmittel, Säure HA und den Katalysator) in ein Reaktionsgefäss, presst Wasserstoff auf und führt die Hydrierung durch. Zweckmässig entspricht das Lösungsmittel dem Alkohol, zum Beispiel Methanol oder Ethanol, der zur Veresterung verwendet wird.

Die Hydrierung kann in verschiedenen Reaktortypen kontinuierlich oder satzweise durch geführt werden. Bevorzugt sind solche Reaktoren, die eine vergleichsweise günstige Durchmischung und gute Wärmeabführung erlauben, wie z. B. Kreislaufreaktoren. Dieser Reaktortyp hat sich insbesondere bei Verwendung geringer Katalysatormengen bewährt.

Zur Isolierung gewünschter Diastereomerer von Tetrahydropterinderivaten, zum Beispiel (6S,αS)-Tetrahydrofolsäure oder -salze, (6S,αS)-Tetrahydrofolsäureester und (6S,αR)-Tetrahydrofolsäureestersalze können bekannte Methoden angewendet werden, zum Beispiel chromatographische Methoden oder fraktionierte Kristallisation, wobei zuvor in an sich bekannter Weise eine Derivatisierung vorgenommen werden kann.. Tetrahydrofolsäureester und Tetrahydrofolsäureestersalze bieten hierbei den Vorteil, dass die Trennung der Diastereomeren erstmals auch mit organischen Lösungsmitteln vorgenommen werden kann und überraschenderweise eine hohe Anreicherung der (6S,αS) bzw. (6S,αR)-Diastereomeren im Kristallisat und der (6R,αS) bzw. (6R,αR)-Diastereomeren in der Mutterlauge beobachtet wird. Aus Tetrahydrofolsäureestem und Tetrahydrofolsäureestersalzen kann die Tetrahydrofolsäure in bekannter Weise durch Hydrolyse gewonnen werden.

Die Isolierung von Tetrahydrofolsäureestem und Tetrahydrofolsäureestersalzen wird aus alkoholischen Reaktionsmedien zweckmässig durch Kristallisation vorgenommen. Es wurde überraschend gefunden, dass (6S,αS)- und (6S,αR)-Diastereomere ausgezeichnet kristallisieren und das Kristallisat sehr hohe Anreicherungen dieser Diastereomeren aufweist. So wurde zum Beispiel bei den Tetrahydrofolsäuredimethylester-sulfonsäureadditionssalzen ein (6S,αS)- zu (6R,αS)-Diastereomerenverhältnis von 99:1 im ersten Kristallisat gefunden. Umgekehrt sind die (6R,αS)- und (6R,αR)-Diastereomeren dann in der Mutterlauge angereichert. Überraschend ist auch, dass das Kristallisat praktisch keinen Katalysator enthält, so dass man die (6S,αS)- und (6S,αR)-Diastereomeren in hoher Reinheit erhält.

Der Grad der optischen Anreicherung kann bei den beschriebenen Verfahren abhängig sein von vorhandenen Zusätzen, vom verwendeten Lösungsmittel, von der Temperatur und von der Konzentration. Durch einfache Versuche können die dem jeweiligen Ziel angepassten optimalen Verfahrensbedingungen ermittelt werden.

Die folgenden Beispiele können mit ähnlichem Erfolg durchgeführt werden durch Ersetzen der generisch oder spezifisch beschriebenen Reaktanden und /oder Verfahrensbedingungen dieser Erfindung durch solche die in den folgenden Beispielen aufgeführt sind. Ebenso sind die folgenden spezifischen Ausführungsformen rein beispielhaft und in keiner Art und Weise limitierend auf den Rest der Offenbarung zu sehen.
Die gesamte Offenbarung aller Anmeldungen, Patente und Publikationen, die in diesem Text zitiert sind, sind durch Referenz miteingeschlossen.

Auf Basis der vorangehenden Beschreibung kann ein Fachmann auf dem Gebiet ohne Weiteres die entscheidenden Elemente der Erfindung entnehmen und ohne vom Grundgedanken und vom Umfang der Erfindung abzuweichen, Änderungen und Ergänzungen anbringen und dadurch die Erfindung an unterschiedliche Bedürfnisse und Bedingungen anpassen.

Die optische Ausbeute beziehungsweise das Verhältnis von (6S,aS)- zum (6R,αS)-Diastereomeren beziehungsweise das Verhältnis (6S,αR)- zum (6R,αR)-Diastereomeren der Tetrahydrofolsäureester und Tetrahydrofolsäureestersalze wird folgendermassen mittels Hochdruckflüssigkeitschromatographie (HPLC) direkt im Reaktionsgemisch bestimmt:
15 mg der Reaktionslösung werden in 1 ml Lösungsmittel gelöst, welches aus 6,8 g β-Cyclodextrin und 270 ml 37% Formaldehyd in 1000 ml Wasser hergestellt wird. Die Trennung erfolgt mittels einer Säule Nucleosil C-8, 5 mm, 240 x 4 mm der Firma Macherey-Nagel und einem Fliessmittel, das folgendermassen hergestellt wird: 6,8 g β-Cyclodextrin werden in einer Mischung aus 8,5 ml Triethylamin, 850 ml Wasser und 150 ml Acetonitril gelöst. Der pH-Wert der Lösung wird durch Zugabe von Essigsäure auf pH = 7,5 eingestellt, und es werden noch 270 ml 37% Formaldehyd zugegeben. Die Detektion der beiden Diastereomeren erfolgt bei einer Wellenlänge von 300 nm.

Für die verwendeten ditertiären Diphosphine werden folgende Abkürzungen verwendet:

### a Hydrierungen im alkoholischen Reaktionsmedium

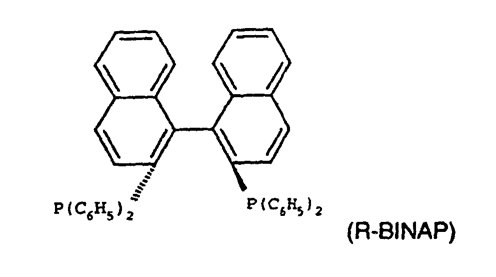

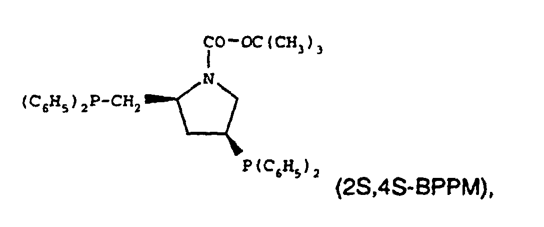

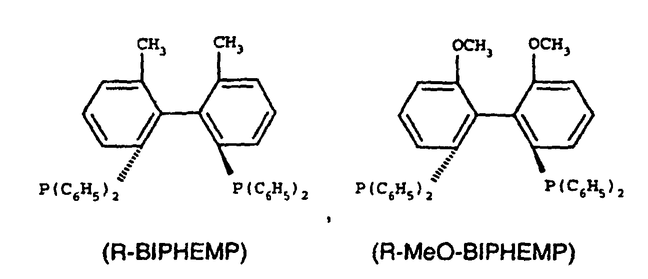

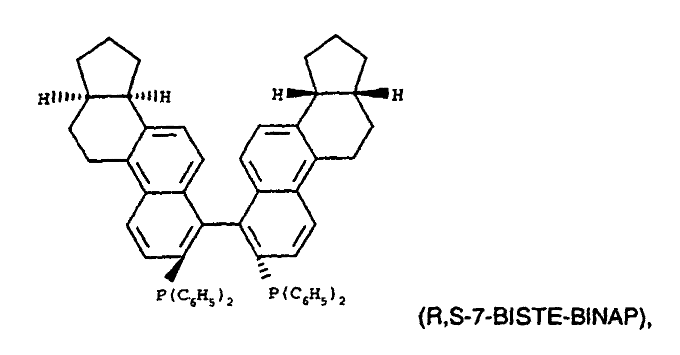

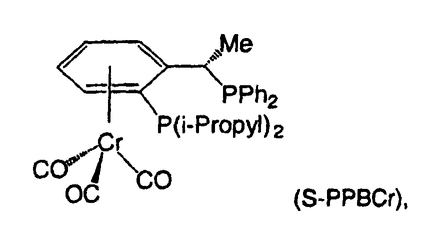

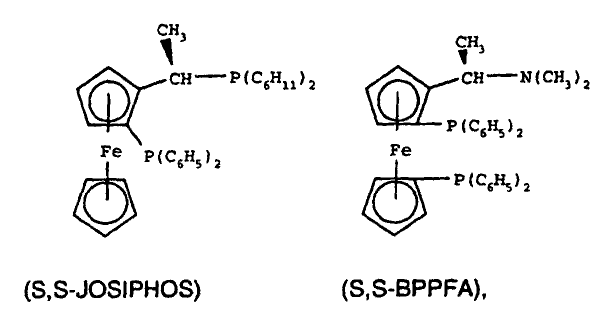

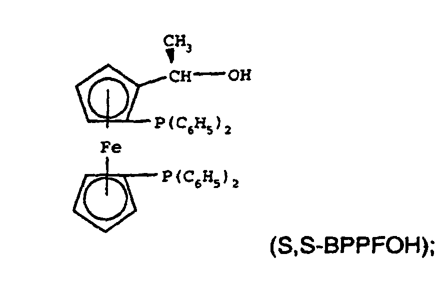

und

### b Hydrierungen im wässrigen Reaktionsmedium

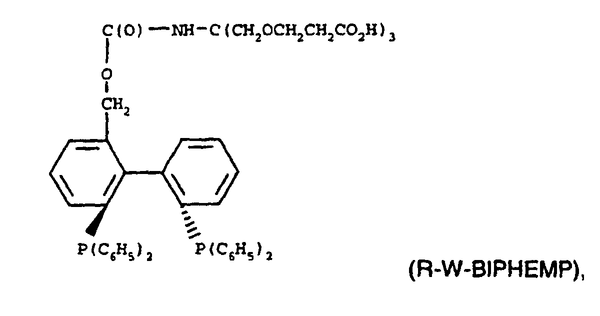

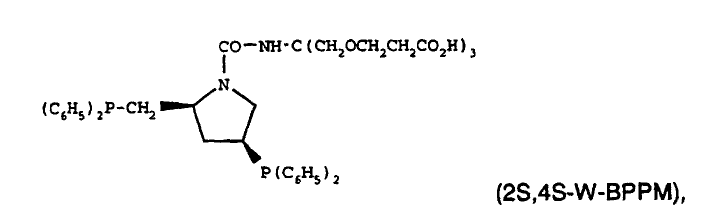

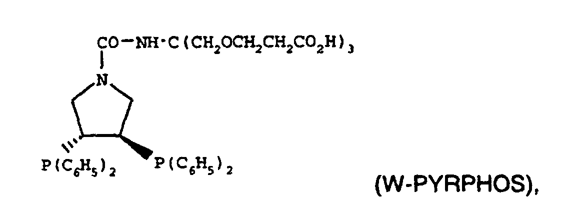

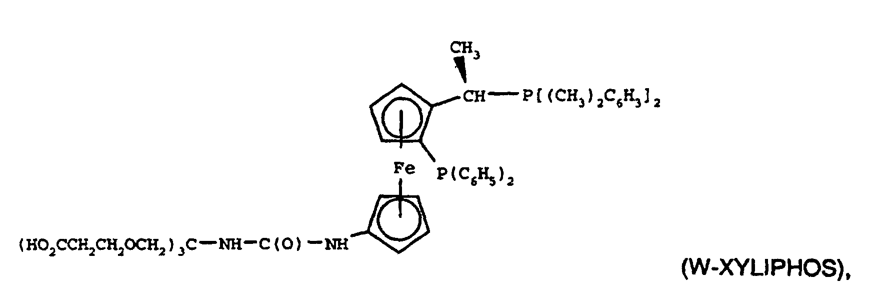

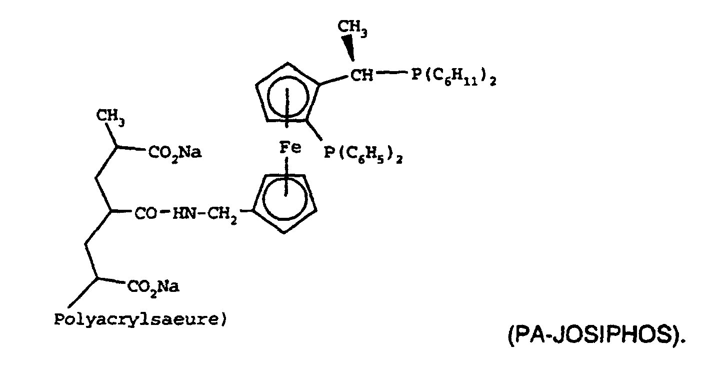

Die Herstellung der Katalysatoren und der Hydrierlösungen, der Transfer von Lösungen und Suspensionen, sowie die Durchführung der Hydrierungen erfolgt unter Ausschluss von Sauerstoff. Dabei kann die dem Fachmann geläufige Schlenktechnik angewendet werden. Es werden entgaste und mit einem Schutzgas, wie zum Beispiel Stickstoff oder Edelgase (Helium, Neon Argon oder Krypton) begaste Lösungsmittel und Autoklaven verwendet. Die Hydrierreaktionen werden in Stahlautoklaven mit Magnetrührer oder Begasungsrührwelle durchgeführt.

### Beispiele

### A Herstellung von Folsäureestersalzen

### Beispiel A1: (αS)-Folsäuredimethylester-benzolsulfonat

800 g (αS)-Folsäuredihydrat (1,68 mol) werden bei 40°C in eine Lösung aus 530 g Benzolsulfonsäure (3,35 mmol) und 20 Litern wasserfreiem Methanol unter einer Stickstoffatmosphäre eingetragen. Das Gemisch wird eine halbe Stunde am Rückfluss erhitzt, abgekühlt und auf ein Volumen von 5 Litern eingeengt. Das abgeschiedene Produkt wird abgenutscht, mit 1 Liter Methanol gewaschen und im Trockenschrank bei 40°C und 20 mbar getrocknet. Man erhält 966 g (αS)-Folsäuredimethylester-benzolsulfonat (1,45 mol, 86% der theoretischen Ausbeute) Das Produkt enthält 26,2% Benzolsulfonsäure, 1,67% Wasser und 2,26% Methanol.
Die Substanz zersetzt sich oberhalb von 150°C.
¹H-NMR in DMSO-d₆: 8.78 (1H, s), 8.46 (2H, bs), 8.32 (1H, d), 7.64-7.68 (m), 7.35-7.40 (m), 6.66 (2H, d), 0.8 (2H, s), 4.39 (1H, m), 3.62 (3H, s), 3.57 (3H, s), 2.42 (2H, m), 1.98-2.11 (2H, m).

### Beispiel A2: (αS)-Folsäurediethylester-benzolsulfonat

8 g (αS)-Folsäuredihydrat (16,76 mmol) werden in eine Lösung aus 3,18 g Benzolsulfonsäure (20,11 mmol) Benzolsutfonsäure und 1,5 Liter wasserfreiem Ethanol eingetragen. Es wird 5 Stunden am Rückfluss erhitzt, auf Raumtemperatur abgekühlt und das abgeschiedene Produkt nach 12 Stunden abgenutscht. Nach dem Trocknen bei 40°C und 20 mbar erhält man 10,09 g (αS)-Folsäuredimethylester-benzolsulfonat (15,29 mmol, 92% der theoretischen Ausbeute, das Produkt enthält 21.8% Benzolsulfonsäure).
Die Substanz zersetzt sich oberhalb von 150°C.
¹H-NMR in DMSO-d6: 8.77 (1H, s), 8.27 (3H, d, bs), 7.66 (m), 7.35 (m), 6.66 (2H, d), 4.59 (2H, s), 4.37 (1H, m), 3.98-4.13 (4H, m), 2.40 (2H, m) 1.97-2.06 (2H, m) 1.06-1.21 (6H, m).

### B Herstellung von wasserlöslichen Ditertiärdiphosphinen

### Beispiel B1: Herstellung von 2S,4S-W-BPPM

### a Herstellung des Triesters

Eine Lösung von 377 mg (0,83 mmol) 2-Diphenylphosphinomethyl-4-diphenylphosphino-pyrrolidin (PPM) in 2,5 ml Toluol wird zu einer Lösung gemäss Beispiel A1 (1,1 mmol Isocyanat-Triester) gegeben und das Gemisch über Nacht gerührt. Nach dem Einengen am Rotationsverdampfer und teilweiser Entfernung des Toluols unter reduziertem Druck wird das Rohprodukt chromatographisch gereinigt (Silikagel: Merck 60; Laufmittel Essigsäureethylester). Es werden 605 mg Produkt erhalten (Ausbeute: 81%).

### b Herstellung der Trisäure

Zu einer Lösung von 590 mg des Triesters gemäss B1a in 5 ml Ethanol werden 1 ml Wasser und 0,6 g KOH gegeben und das Gemisch während 3 Stunden gerührt. Dann wird bei reduziertem Druck das Ethanol abgedampft und das Gemisch in 25 ml Wasser gelöst. Die Lösung wird anschliessend mit 2 n HCl sauer gestellt und mehrmals mit Essigsäureethylester extrahiert. Die organischen Phasen werden gesammelt, mit Wasser gewaschen, über Natriumsulfat getrocknet und schliesslich bis zur Trockne unter reduziertem Druck eingedampft. Das Produkt wird als weisser Feststoff in 88% Ausbeute erhalten.

### Beispiel B2: Herstellung von W-PYRPHOS

### a Herstellung des Triesters

Es wird wie in Beispiel B1a verfahren, aber 3,4-Diphenylphosphino-pyrrolidin (Pyrphos) als Ausgangsverbindung verwendet. Das Reaktionsprodukt wird in einer Ausbeute von 63% erhalten.

### b Herstellung der Trisäure

Es wird wie in Beispiel B1b verfahren. Das Produkt wird als weisser Feststoff in 95% Ausbeute erhalten.

### Beispiel B3: Herstellung von R-W-BIPHEMP

### a Herstellung des Triesters

Es wird wie in Beispiel B1a verfahren, aber 2,2'-Diphenylphosphino-5-methyl-5'hydroxymethyl (HO-Biphemp) als Ausgangsverbindung verwendet. Das Reaktionsprodukt wird in einer Ausbeute von 82% erhalten.

### b Herstellung der Trisäure

Es wird wie in Beispiel B1b verfahren. Das Produkt wird als weisser Feststoff in 92% Ausbeute erhalten.

### Beispiel B4: Herstellung von W-XYLIPHOS

### a Herstellung des Triesters

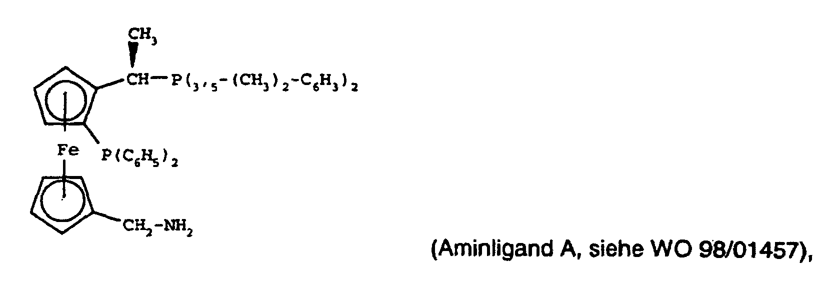

Eine Lösung von 1 g (1,5 mMol) Aminligand A in 8 ml Methylenchlorid wird bei 0°C zu einer äquimolaren Menge Carbonyldiimidazol in 6 ml Methylenchlorid gegeben und das Reaktionsgemisch anschliessend 2 Stunden bei Raumtemperatur gerührt. Dann werden 1,6 Äquivalente H₂N-C(CH₂-O-CH₂CH₂C(O)-OCH₂CH₃ und 5 mg Dibutylzinn-dilaurat zugegeben und das Gemisch 48 Stunden bei 50°C gerührt. Nach chromatographischer Reinigung (Silikagel: Merck 60; Laufmittel: Hexan/Essigsäureethylester, 1:1) wird das Produkt als fast festes, oranges Öl in 65% Ausbeute erhalten.

### b Herstellung der Trisäure:

1 g Diphosphin-Triester wird in 10 ml Ethanol gelöst und mit 1 ml 20%-iger wässeriger KOH-Lösung versetzt. Nach 2 Stunden Rühren wird das Ethanol unter reduziertem Druck abgedampft und das Produkt in 20 ml Wasser gelöst. Durch Zugabe von 2 n HCl wird das Produkt gefällt, abfiltriert, mehrmals mit Wasser gewaschen und schliesslich bei 50°C am Hochvakuum getrocknet. Das Produkt wird als orange-gelber Feststoff in einer Ausbeute von 92% erhalten.

### Beispiel B5: Herstellung von PA-JOSIPHOS

Der Ligand wird gemäss Beispiel B25 der WO 98/01457 hergestellt. MW: 1480.

### C Hydrierungen in alkoholischem Reaktionsmedium

### Beispiele C1-C29:

### Methode A

6,72 mg [Ir(COD)Cl]₂ (10 µmol) und Diphosphosphin-Ligand (25 µmol) werden eingewogen, entgast und in Dichlormethan gelöst. Dichlormethan wird im Hochvakuum abkondensiert und der Rückstand in 5 ml Methanol aufgenommen. 1,25 g (αS)-Folsäuredimethylester-benzolsulfonat gemäss Beispiel A1 (2 mmol) werden in 25 ml Methanol suspendiert und zum Katalysator gegeben. Die Suspension wird im Stickstoffgegenstrom in einen 100 ml Autoklaven gegeben und so lange hydriert, bis keine Wasserstoffaufnahme mehr erfolgt. COD steht für Cyclooctadien.

### Methode B

8,12 mg [Rh(COD)₂]BF₄ (20 µmol) und Diphosphin-Ligand (25 µmol) werden eingewogen, entgast und in einer Mischung aus Tetrahydrofuran und Methanol gelöst. Die Lösungsmittel werden im Hochvakuum abkondensiert und der Rückstand in 5 ml Methanol aufgenommen. 1,25 g (αS)-Folsäuredimethylester-benzolsulfonat gemäss Beispiel A1 (2 mmol) werden in 25 ml Methanol suspendiert und zum Katalysator gegeben. Die Suspension wird im Stickstoffgegenstrom in einen 100 ml Autoklaven gegeben und so lange hydriert, bis keine Wasserstoffaufnahme mehr erfolgt.

Die Hydrierungen werden bei einer Temperatur von 70°C (25°C in Beispiel C9) und bei einem Druck von 80 bar (20 bar in Beispielen C9 und C10) durchgeführt. Die Ergebnisse sind in Tabelle 1 angegeben.

### Beispiel C25:

28,79 g (αS)-Folsäure-dihydat (60 mmol) werden in einen 1 I Autoklaven eingewogen und entgast. 121,82 mg [Rh(COD)₂]BF₄ (300 µmol) und 233,51 mg R-BINAP (375 µmol) werden eingewogen, entgast und in einer Mischung aus Tetrahydrofuran und Methanol gelöst. Die Lösungsmittel werden im Hochvakuum abkondensiert und der Rückstand wird in 50 ml Methanol aufgenommen. 9,49 g wasserfreie Benzolsulfonsäure (60 mmol) werden in 200 ml Methanol gelöst und im Stickstoffgegenstrom in den Autoklaven gegeben. Man gibt noch weitere 550 ml Methanol hinzu, sowie die Katalysatorlösung. Es wird bei 70°C und 20 bar Wasserstoffdruck 15 Stunden lang hydriert. Der Umsatz zu Tetrahydrofolsäuredimethylester-benzolsulfonat beträgt 80%. Das Verhältnis der Diastereomeren (6S,αS):(6R,αS) beträgt 71:28.

### Beispiel C26:

16,68 mg Ru(BINAP)(2-methylallyl)₂ (20 µmol) (hergestellt nach J.P. Genet et al, Tetrahedron Asymmetry, Vol. 5, No. 4, pp.665-674, 1994) werden in 5 ml eritgastem Methanol suspendiert und mit einer Suspension von 1,25 g (αS)-Folsäuredimethylester-benzolsulfonat gemäss Beispiel A1 (2 mmol) in 25 ml Methanol versetzt. Die Suspension wird im Stickstoffgegenstrom in einen 100 ml Autoklaven überführt und 17 Stunden bei 70°C und 80 bar Wasserstoffdruck hydriert. Der Umsatz zu Tetrahydrofolsäuredimethylester-benzolsulfonat beträgt 30%. Das Verhältnis der Diastereomeren (6S,αS):(6R,αS) beträgt 62:37.

### Beispiel C27:

8,12 mg [Rh(COD)₂]BF₄ (20 µmol) und 15,57 mg BINAP (25 µmol) werden eingewogen, entgast und in einer Mischung aus Tetrahydrofuran und Methanol gelöst. Die Lösungsmittel werden im Hochvakuum abkondensiert und der Rückstand in 5 ml Methanol aufgenommen. Zum Katalysator wird eine Suspension aus 0,39 g 6-Hydroxymethylpterin (2 mmol) (hergestellt nach P.H. Boyle et al., Chem. Ber.; Bd. 113, Seite 1514, 1980) und 0,32 g Benzolsulfonsäure (2 mmol) in 25 ml Methanol gegeben. Die Mischung wird im Stickstoffgegenstrom in einen 100 ml Autoklaven gegeben und bei 70°C und 80 bar Wasserstoffdruck 15 Stunden lang hydriert. Der Umsatz zu 6-Hydroxymethyl-5,6,7,8-tetrahydropterin beträgt 85% und wird mit HPLC direkt aus der Reaktionslösung bestimmt. Die verwendete HPLC-Methode ist die gleiche, die für die quantitative Bestimmung der Tetrahydrofolsäure verwendet wird.

### Beispiel C28:

8,12 mg [Rh(COD)₂]BF₄ (20 µmol) und 15,57 mg BINAP (25 µmol) werden eingewogen, entgast und in einer Mischung aus Tetrahydrofuran und Methanol gelöst. Die Lösungsmittel werden im Hochvakuum abkondensiert und der Rückstand in 5 ml Methanol aufgenommen. Zum Katalysator wird eine Suspension aus 0,48 g 6-Phenylpterin (2 mmol) (hergestellt nach H. Yamamoto et al., Chem. Ber., Bd. 106, Seite 3175, 1973) und 0,32 g Benzolsulfonsäure (2 mmol) in 25 ml Methanol gegeben. Die Mischung wird im Stickstoffgegenstrom in einen 100 ml Autoklaven gegeben und bei 70°C und 80 bar Wasserstoffdruck 15 Stunden lang hydriert. Der Umsatz zu 6-Phenyl-5,6,7,8-tetrahydropterin beträgt 64% und wird mit HPLC direkt aus der Reaktionslösung bestimmt. Die verwendete HPLC-Methode ist die Gleiche, die für die quantitative Bestimmung der Tetrahydrofolsäure verwendet wird.

### Beispiel C29:

8,12 mg [Rh(COD)₂]BF₄ (20 µmol) und 15,57 mg BINAP (25 µmol) werden eingewogen, entgast und in einer Mischung aus Tetrahydrofuran und Methanol gelöst. Die Lösungsmittel werden im Hochvakuum abkondensiert und der Rückstand in 5 ml Methanol aufgenommen. Zum Katalysator wird eine Suspension aus 0,35 g 6-Methylpterin (2 mmol) (hergestellt nach P. Waring et al., Aust. J. Chem., Bd. 38, Seite 629, 1985) und 0,32 g Benzolsulfonsäure (2 mmol) in 25 ml Methanol gegeben. Die Mischung wird im Stickstoffgegenstrom in einen 100 ml Autoklaven gegeben und bei 70°C und 80 bar Wasserstoffdruck 15 Stunden lang hydriert. Der Umsatz zu 6-Methyl-5,6,7,8-tetrahydropterin beträgt 63% und wird mit HPLC direkt aus der Reaktionslösung bestimmt. Die verwendete HPLC-Methode ist die Gleiche, die für die quantitative Bestimmung der Tetrahydrofolsäure verwendet wird.

**Tabelle 1:**

| Beispiel | Metall | Zusatz | Ligand | S/C | Lösungsmittel | Umsatz | Verhältnis (6S,αS): (6R,αS) | Methode |
|---|---|---|---|---|---|---|---|---|
| C1 | Ir | - | R-BINAP | 100 | MeOH | 80 % | 65 :35 | A |
| C2 | Ir | Bu₄Nl | (2S,4S)-BPPM | 100 | MeOH | 80% | 62:38 | A¹⁾ |
| C3 | Ir | LiCl | (2S,4S)-BPPM | 100 | MeOH | 90 % | 30 :70 | A²⁾ |
| C4 | Ir | - | S,S-BPPFA | 100 | MeOH | 60 % | 67:33 | A |
| C5 | Rh | - | R-BINAP | 100 | MeOH | 72% | 74:26 | B |
| C6 | Rh | Nal | R-BINAP | 100 | MeOH | 85% | 67:33 | B³⁾ |
| C7 | Rh | - | R-BINAP | 100 | MeOH | 70% | 71:29 | B⁴⁾ |
| C8 | Rh | - | R-BINAP | 100 | EtOH | 80 % | 76:24 | B⁵⁾ |
| C9 | Rh | - | R-BINAP | 100 | i-PrOH | 20 % | 80:20 | B⁶⁾ |
| C10 | Rh | - | R-BINAP | 100 | 1,2-Propandiol | 62% | 75:25 | B⁷⁾ |
| C11 | Rh | | R-BINAP | 100 | Ethylengly kol | 56% | 78:22 | B⁸⁾ |
| C12 | Rh | - | R-BINAP | 100 | MeOH | 90 % | 73:27 | B |
| C13 | Rh | - | R-BINAP | 200 | MeOH | 90 % | 72:28 | B⁹⁾ |
| C14 | Rh | - | R-BINAP | 100 | MeOH/ THF/ 1:1 | 90 % | 72:28 | B¹⁰⁾ |
| C15 | Rh | - | R-BINAP | 700 | MeOH | 60 % | 69:31 | B¹¹⁾ |
| C16 | Rh | - | S-PPBCr | 100 | MeOH | 70% | 71:29 | B |
| C17 | Rh | - | S,S-BPPFOH | 100 | MeOH | 90 % | 58:42 | B |
| C18 | Rh | - | (2S,4S)-BPPM | 100 | MeOH | 90 % | 68:32 | B |
| C19 | Rh | - | S,S-JOSIPHOS | 100 | MeOH | 60% | 61:39 | B |
| C20 | Rh | - | R-BIPHEMP | 100 | MeOH | 80 % | 71:29 | B |
| C21 | Rh | - | R-MeO-BIPHEP | 100 | MeOH | 80 % | 69:31 | B |
| C22 | Rh | - | R,S-7-BISTE-BINAP | 100 | MeOH | 90 % | 71:29 | B |
| C23 | Ir | Pa | R-BINAP | 100 | MeOH/ THF, 1:1 | 90% | 72:28 | A¹²⁾ |
| C24 | Rh | - | 1,2-Bis(diphenylphosphino)ethan | 100 | MeOH | 90 % | 51:49 | B |
| Legende: | | | | | | | | |
| Bu steht für Butyl, MeOH für Methanol, EtOH für Methanol, i-PrOH für Isopropanol und THF für Tetrahydrofuran, Pa für Parabansäure. | | | | | | | | |

| | | | | | | | | |
|---|---|---|---|---|---|---|---|---|
| ¹⁾ Bei diesem Experiment gibt man 73,9 mg Tetrabutylammoniumjodid (0,2 mmol) zum Katalysator. | | | | | | | | |
| ²⁾ Bei diesem Experiment gibt man 8,48 mg Lithiumchlorid (0,2 mmol) zum Katalysator. | | | | | | | | |
| ³⁾ Bei diesem Experiment gibt man 29,98 mg Natriumiodid (0,2 mmol) zum Katalysator. | | | | | | | | |
| ⁴⁾ Bei diesem Experiment werden 3,55 g (αS)-Folsäurediemthylester-benzolsulfonat (5,66 mmol) nach Methode B in den angegebenen Lösungsmittelvolumina umgesetzt, so dass die Substratkonzentration 15% beträgt. | | | | | | | | |
| ⁵⁾ Bei diesem Experiment wurden 1,31 g (αS)-Folsäurediethylester-benzolsulfonat (2 mmol) nach Methode B in Ethanol zu Tetrahydrofolsäurediethylester-benzolulfonat umgesetzt. | | | | | | | | |
| ⁶⁾ Bei diesem Experiment wird die Hydrierung des Folsäuredimethylester-benzolsulfonates in 30 ml i-Propanol durchgeführt. | | | | | | | | |
| ⁷⁾ Bei diesem Experiment wird die Hydrierung des Folsäuredimethylester-benzolsulfonates in 30 ml 1,2-Propandiol durchgeführt. | | | | | | | | |
| ⁸⁾ Bei diesem Experiment wird die Hydrierung des Folsäuredimethylester-benzolsulfonates in 30 ml Ethylenglykol durchgeführt. | | | | | | | | |
| ⁹⁾ Bei diesem Experiment wird der Katalysator aus 4,06 mg [Rh(COD)₂]BF₄ (10 µmol) und 7,78 mg R-BINAP (12,5 µmol) hergestellt. | | | | | | | | |
| ¹⁰⁾ Bei diesem Experiment wird die Hydrierung in einem Gemisch aus 15 ml THF und 15 ml MeOH durchgeführt. | | | | | | | | |
| ¹¹⁾ Bei diesem Experiment wird der Katalysator aus 1,16 mg [Rh(COD)₂]BF₄ (2,86 µmol) und 2,22 mg R-BINAP (3,57 µmol) hergestellt. | | | | | | | | |
| ¹²⁾ Bei diesem Experiment gibt man 4,56 mg Parabansäure (40 mmol) zum Katalysator. Die Hydrierung wird in MeOH / THF (1:1) durchgeführt. | | | | | | | | |

### D Hydrierungen in wässrigem Reaktionsmedium

### Beispiele D1-D8:

0,0025 mmol Ligand werden in 5 ml Wasser und 0,5 ml Puffer pH 7 (0,041 Mol Na₂HPO₄ und 0,028 mmol KH₂PO₄ in 1 I Wasser) gelöst. Die Carbonsäuregruppen der Liganden werden dann mit 0,1N NaOH umgesetzt, bis eine klare Lösung entsteht. Die resultierende Lösung wird zu 7,4 mg (0,02 mmol) [Rh(NBD)₂]BF₄ gegeben und gerührt, bis sich eine Lösung gebildet hat (NBD ist Norbornadien). Diese Lösung wird zu einer Lösung von 2 mmol (αS)-Folsäuredinatriumsalz in 11 ml Wasser und 1,5 ml Puffer pH 7 gegeben und die Mischung mit Hilfe einer Kanüle im Argongegenstrom in einen Hydrierautoklaven mit Begasungsrührer übergeführt. Der Autoklav wird verschlossen, das Argon gegen Wasserstoff ausgetauscht, und schliesslich Wasserstoff bis auf den gewünschten Druck aufgepresst. Der Wasserstoffdruck wird über ein Reduzierventil aus dem Vorratsgefäss aufrechterhalten. Die Hydrierung wird durch Einschalten des Rührers gestartet. In der nachfolgenden Tabelle 2 ist als Hydrierzeit der Stillstand der Reaktion (keine Wasserstoffaufnahme mehr) angegeben. Falls nicht anders angegeben, entspricht das dem vollständigen Umsatz der (αS)-Folsäure. Der Druck beträgt 80 bar und die Reaktionstemperatur 70°C (30°C in Beispiel D6). Das molare Verhältnis von Substrat zu Katalysator (S/C) beträgt in den Beispielen D1-D7 100 und in Beispiel D8 1000. Die Ergebnisse sind in Tabelle 2 zusammengefasst.

**Tabelle 2:**

| Beispiel Nr. | Ligand | Zeit (Stunden) | Verhältnis (6S,αS):( αR, αS) | Bemerkungen |
|---|---|---|---|---|
| D1 | (S,R)-PA-JOSIPHOS | 17,5 | 68:32 | 25% (αS)-Folsäure |
| D2 | (2S,4S)-W-BPPM | 4 | 73,4:27,6 | |
| D3 | (3R,4R)-PYRPHOS | 2 | 59:41 | |
| D4 | (R)-W-BIPHEMP | 3,2 | 73:27 | |
| D5 | (S,R)-W-XYLIPHOS | 0,5 | 66:34 | |
| D6 | (S,R)-W-XYLIPHOS | 12 | 74,4:25,6 | |
| D7 | (S,R)-W-XYLIPHOS | 0,6 | 68:32 | Hydrierung von (αS)-Folsäure-Suspension bei pH 6,3¹⁾ |
| D8 | (S,R)-W-XYLIPHOS | 4 | 65:35 | S/C 1000²⁾ |

| | | | | |
|---|---|---|---|---|
| Legende: ¹⁾ Puffer pH 6: 0,01 Mol Na₂HPO₄ und 0,071 Mol KH₂PO₄ in 1l Wasser; bei Reaktionsende werden nochmals 4 ml 1N KH₂PO₄ zugegeben. | | | | |
| ²⁾ Es werden 5 mmol (αS)-Folsäuredinatriumsalz, 0,005 mmol [Rh(NBD)₂]BF₄ und 0,00675 mmol Ligand in gesamt 16 ml Wasser und 2 ml Puffer pH 7 eingesetzt. | | | | |

### E Isolierung von Tetrahydrofolsäuredimethylester-benzolsulfonat und Tetrahydrofolsäure-benzolsulfonat

### Beispiel E1: Aus Reaktion C1

### a Isolierung von Tetrahydrofolsäuredimethylester-benzolsulfonat

Die Reaktionslösung aus Reaktion C1 wird unter Ausschluss von Sauerstoff auf 1/6 des Volumens eingeengt. Die so erhaltene Suspension wird unter Stickstoffatmosphäre für 2 Stunden bei 4°C gelagert, das abgeschiedene Produkt wird abgesaugt, mit wenig kaltem Methanol gewaschen und bei 40°C und 20 mbar getrocknet. Man erhält 0,55 g Tetrahydrofolsäuredimethylester-benzolsulfonat (0,87 mmol, 44% der theoretischen Ausbeute). Das Verhältnis der Diastereomeren des Tetrahydrofolsäuredimethylester-benzolsulfonates (6S,αS):(6R,αS) beträgt 99:1, bestimmt mittels HPLC. [a]₅₈₉ = -69.8° (c = 1 in Dimethylsulfoxid).
Die Substanz zersetzt sich oberhalb von 150°C.
¹H-NMR in DMSO-d₆: 10.61 (1H, bs), 8.35 (1H, d), 7.6-7.74 (m), 7.51 (1H, bs), 7.30-7.37 (m), 6.70 (2H, d, 2H, bs), 4.42 (2H, m), 3.63 (3H, s), 3.58 (3H, s), 3.50 (1H, m), 3.38 (1H, m), 3.28 (1H, m), 2.44 (2H, m), 2.01-2.13 (2H, m)

### b Hydrolyse des Tetrahydrofolsäurediemthylester-benzolsulfonates:

0,55 g Tetrahydrofolsäuredimethylester-benzolsulfonat [(6S,αS):(6R,αS) = 99:1] (0,87 mmol) und 0,32 g Natriumcarbonat (3,02 mmol) werden unter Ausschluss von Sauerstoff in 4 ml Wasser gelöst. Man erhitzt auf 85°C und stellt nach 30 Minuten den pH-Wert mit 37%iger Salzsäure auf pH = 7,5 ein. Bei 75°C werden 0,2 g Benzolsulfonsäure in 0,6 ml Wasser zugegeben und anschliessend wird der pH-Wert mit 37%iger Salzsäure auf pH = 0,8 eingestellt. Man lässt die Lösung auf Raumtemperatur abkühlen und rührt noch drei Stunden. Das Produkt wird abgenutscht und im Trockenschrank bei 30°C und 20 mbar 4 Tage lang getrocknet. Man erhält 8,4g Tetrahydrofolsäure-benzolsulfonat (13,92 mmol, 88% der theoretischen Ausbeute).
Das Diastereomerenverhältnis Tetrahydrofolsäure-benzolsulfonates (6S,αS):(6R,αS) beträgt 99:1. Die Bestimmungsmethode ist in der EP-0 495 204 beschrieben.
Die Eigenschaften des Tetrahydrofolsäure-benzolsulfonates sind mit denen des in EP-0 495 204 B1 beschriebenen Produktes identisch.

## Patentansprüche

1. Verfahren zur Herstellung von Tetrahydropterin und Tetrahydropterinderivaten durch Hydrierung Pterin und Pterinderivaten mit Wasserstoff in Gegenwart eines Hydrierkatalysators, **dadurch gekennzeichnet, dass** man die Hydrierung in einem polaren Reaktionsmedium durchführt und in dem Reaktionsmedium lösliche d-8-Metallkomplexe als Hydrierkatalysatoren verwendet, die tertiäre Phosphine, tertiäre Phosphane als Liganden, bidentate Liganden mit tertiären Aminogruppen und tertiären Phosphingruppen, oder bidentate Liganden mit zwei tertiären Phosphingruppen enthalten, wobei bidentate Liganden mit dem Metallatom einen 5- bis 10-gliedrigen Ring bilden.

2. Verfahren gemäss Anspruch 1, **dadurch gekennzeichnet, dass** das polare Reaktionsmedium ein wässriges oder alkoholisches Reaktionsmedium ist.

3. Verfahren gemäss Anspruch 1, **dadurch gekennzeichnet, dass** man als Pterinderivate Folsäure, Folsäuresalze, Folsäureester, Folsäureestersalze oder deren Dihydroformen zur Hydrierung mit Wasserstoff in Gegenwart eines Hydrierkatalysators verwendet, die Hydrierung bei erhöhtem Druck in Gegenwart von im Reaktionsmedium gelösten Metallkomplexen als Hydrierkatalysatoren durchführt, mit der Massgabe, dass bei Verwendung von Folsäure, deren Carbonsäuresalzen oder deren Dihydroformen ein wässriges, sowie bei Verwendung von Folsäureestern, Folsäureestersalzen oder deren Dihydroformen ein alkoholisches Reaktionsmedium vorliegt.

4. Verfahren gemäss Anspruch 1 zur asymmetrischen Hydrierung von prochiralen Pterinderivaten mit Wasserstoff in Gegenwart eines Hydrierkatalysators, **dadurch gekennzeichnet, dass** man die Hydrierung in einem polaren Reaktionsmedium durchführt und in dem Reaktionsmedium lösliche Metallkomplexe als Hydrierkatalysatoren verwendet, wobei die Metallkomplexe chirale Liganden enthalten.

5. Verfahren gemäss Anspruch 4 zur asymmetrischen Hydrierung von Folsäure, Folsäuresalzen, Folsäureestern, Folsäureestersalzen oder deren Dihydroformen als Pterinderivate, mit Wasserstoff in Gegenwart eines Hydrierkatalysators, **dadurch gekennzeichnet, dass** man die Hydrierung bei erhöhtem Druck in Gegenwart von im Reaktionsmedium gelösten Metallkomplexen als Hydrierkatalysatoren durchführt, wobei die Metallkomplexe chirale Liganden enthalten, mit der Mass gabe, dass bei Verwendung von Folsäure, deren Carbonsäuresalzen oder deren Dihydroformen ein wässriges, sowie bei Verwendung von Folsäureestern, Folsäureestersalzen oder deren Dihydroformen ein alkoholisches Reaktionsmedium vorliegt.

6. Verfahren gemäss Anspruch 5, **dadurch gekennzeichnet, dass** die Folsäureestersalze der Formel III entsprechen und in Form ihrer Enantiomeren oder Mischungen vorliegen, 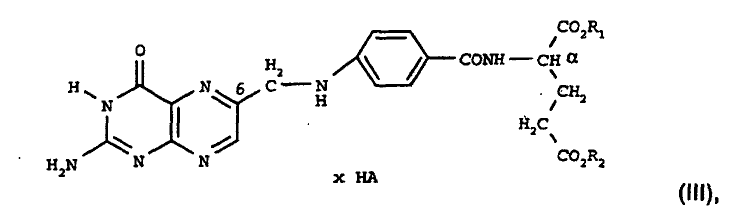 worin R₁ oder R₂ H ist, und R₂ oder R₁, oder beide R₁ und R₂ unabhängig voneinander einen monovalenten Kohlenwasserstoffrest oder einen über ein C-Atom gebundenen Heterokohlenwasserstoffrest mit Heteroatomen ausgewählt aus der Gruppe -O-, -S- und -N- darstellen,
HA für eine ein- bis dreibasischen anorganische oder organische Säure steht, und x eine ganze Zahl von 1 bis 6 oder eine gebrochene Zahl zwischen 0 und 6 bedeutet.

7. Verfahren gemäss Anspruch 6, **dadurch gekennzeichnet, dass** die Säure HA in Formel III unsubstituierte oder substituierte Phenylsulfonsäure ist.

8. Verfahren gemäss Anspruch 1, **dadurch gekennzeichnet, dass** es unter einem Wasserstoffdruck von 1 bis 500 bar durchgeführt wird.

9. Verfahren gemäss Anspruch 1, **dadurch gekennzeichnet, dass** die Reaktionstemperatur 0 bis 150°C beträgt.

10. Verfahren gemäss Anspruch 1, **dadurch gekennzeichnet, dass** das molare Verhältnis von Substrat zu Katalysator 10 bis 100000 beträgt.

11. Verfahren gemäss Anspruch 1, **dadurch gekennzeichnet, dass** das wässrige Reaktionsmedium Wasser oder Wasser in Abmischung mit einem organischen Lösungsmittel ist.

12. Verfahren gemäss Anspruch 1, **dadurch gekennzeichnet, dass** das alkoholische Reaktionsmedium ein Alkohol, oder ein Alkohol in Abmischung mit einem organischen Lösungsmittel ist.

13. Verfahren gemäss Anspruch 1, **dadurch gekennzeichnet, dass** die Metallkomplexe ein d-8 Metall, bevorzugt Iridium, Rhodium oder Ruthenium enthalten.

14. Verfahren gemäss Anspruch 1, **dadurch gekennzeichnet, dass** der Metallkomplex achirale oder chirale ditertiäre Diphosphine als Liganden enthält.

15. Verfahren gemäss Anspruch 14, **dadurch gekennzeichnet, dass** es sich bei den ditertiären Diphosphinen für ein alkoholisches Reaktionsmedium um solche handelt, bei denen die Phosphingruppen (a) an verschiedene C-Atome einer Kohlenstoffkette mit 2 bis 4 C-Atomen, oder (b) direkt oder über eine Brückengruppe -CRₐR_{b}- in den Orthostellungen eines Cyclopentadienylrings oder an je einen Cyclopentadienyl eines Ferrocenyls gebunden sind, wobei Rₐ und R_{b} gleich oder verschieden sind und für H, C₁-C₈-Alkyl, C₁-C₄-Fluoralkyl, C₅-C₆-Cycloalkyl, Phenyl, Benzyl, oder mit 1 bis 3 C₁-C₄-Alkyl oder C₁-C₄-Alkoxy substituiertes Phenyl oder Benzyl stehen.

16. Verfahren gemäss Anspruch 15, **dadurch gekennzeichnet, dass** die Diphosphine für ein alkoholisches Reaktionsmedium der Formel IV entsprechen,
R₄R₅P-R₆-PR₇R₈ (IV),
worin
R₄, R₅, R₇ und R₈ unabhängig voneinander einen Kohlenwasserstoffrest mit 1 bis 20 C-Atomen darstellen, die unsubstituiert oder substituiert sind mit Halogen, C₁-C₆-Alkyl, C₁-C₆-Halogenalkyl, C₁-C₆-Alkoxy, C₁-C₆-Halogenalkoxy, (C₆H₅)₃Si, (C₁-C₁2-Alkyl)₃Si, -NH₂, -NH(C₁-C₁₂-Alkyl), -NH(Phenyl), -NH(Benzyl), -N(C₁-C₁₂-Alkyl)₂, -N (Phenyl)₂, -N(Benzyl)₂, Morpholinyl, Piperidinyl, Pyrrolidinyl, Piperazinyl, -Ammonium-X₃⁻, -SO₃M₁, -CO₂M₁, -PO₃M₁, oder -CO₂-C₁-C₆-Alkyl, worin M₁ ein Alkalimetall oder Wasserstoff darstellt, und X₃⁻ das Anion einer einbasischen Säure ist; oder R₄ und R₅ sowie R₇ und R₈ je zusammen unsubstiuiertes oder mit Halogen, C₁-C₆-Alkyl oder C₁-_{C6}-Alkoxy substituiertes Tetramethylen, Pentamethylen oder 3-Oxa-pentan-1,5-diyl bedeuten; und
R₆ unsubstituiertes oder mit C₁-C₆-Alkyl, C₁-C₆-Alkoxy, C₅- oder C₆-Cycloalkyl, Phenyl, Naphthyl oder Benzyl substituiertes C₂-C₄-Alkylen; unsubstituiertes oder mit C₁-C₆-Alkyl, Phenyl oder Benzyl substituiertes 1,2- oder 1,3-Cycloalkylen, 1,2-oder 1,3-Cycloalkenylen, 1,2- oder 1,3-Bicycloalkylen oder 1,2- oder 1,3-Bicycloalkenylen mit 4 bis 10 C-Atomen; unsubstituiertes oder mit C₁-C₆-Alkyl, Phenyl oder Benzyl substituiertes 1,2- oder 1,3-Cycloalkylen, 1,2- oder 1,3-Cycloalkenylen, 1,2- oder 1,3-Bicycloalkylen oder 1,2- oder 1,3-Bicycloalkenylen mit 4 bis 10 C-Atomen, an deren 1- und/oder 2-Stellungen oder an deren 3-Stellung Methylen oder C₂-C₄-Alkyliden gebunden ist; in den 2,3-Stellungen mit R₉R₁₀C(O-)₂ substituiertes 1,4-Butylen, das in den 1- und/oder 4-Stellungen unsubstituiert oder mit C₁-C₆-Alkyl, Phenyl oder Benzyl substituiert ist, und wobei R₉ und R₁₀ unabhängig voneinander Wasserstoff, C₁-C₆-Alkyl, Phenyl oder Benzyl darstellt; 3,4- oder 2,4-Pyrrolidinylen oder Methylen-4-pyrrolidin-4-yl, deren N-Atom substituiert ist mit Wasserstoff, C₁-C₁₂-Alkyl, Phenyl, Benzyl, C₁-C₁₂-Alkoxycarbonyl, C₁-C₈-Acyl, C₁-C₁₂-Alkylaminocarbonyl; oder unsubstituiertes oder mit Halogen, -OH, C₁-C₆-Alkyl, C₁-C₆-Alkoxy, Phenyl, Benzyl, Phenyloxy oder Benzyloxy substituiertes 1,2-Phenylen, 2-Benzylen, 1,2-Xylylen, 1,8-Naphthylen, 2,2'-Dinaphthylen oder 2,2'-Diphenylen bedeutet; oder R₆ für einen Rest der Formeln 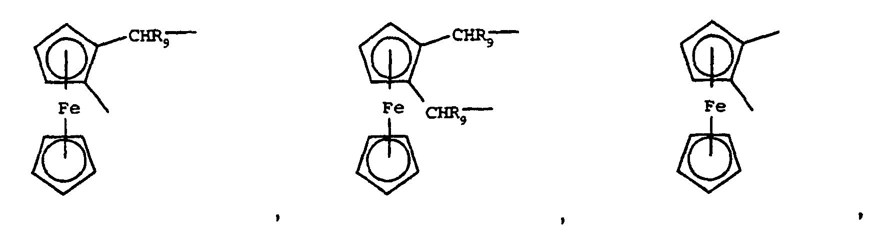 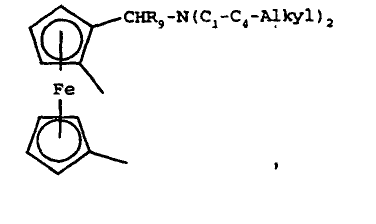 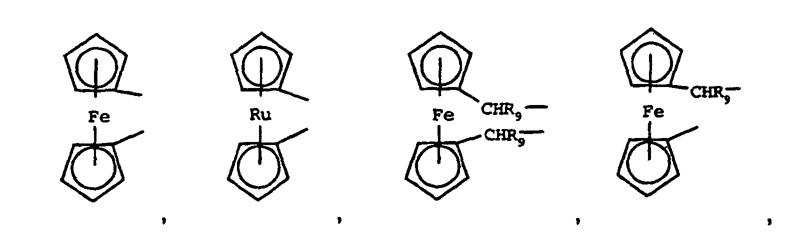 steht, worin R₉ Wasserstoff, C₁-C₈-Alkyl, C₁-C₄-Fluoralkyl, unsubstituiertes oder mit 1 bis 3 F, Cl, Br, C₁-C₄-Alkyl, C₁-C₄-Alkoxy oder Fluormethyl substituiertes Phenyl bedeutet.

17. Verfahren gemäss Anspruch 14, **dadurch gekennzeichnet, dass** Diphosphine für ein wässriges Reaktionsmedium solche sind, die eine oder mehrere wasser-solubilisierende polare Substituenten enthalten, die entweder direkt oder über eine Brückengruppe an Substituenten der Phosphingruppen und/oder an das Gerüst des Diphosphins gebunden sind.

18. Verfahren gemäss Anspruch 17, **dadurch gekennzeichnet, dass** Diphosphine für ein wässriges Reaktionsmedium solche der Formel XLIII sind,
(M₁O₂C-CH₂CH₂-O-CH₂)₃C-NR₄₂-CO―R₄₁ (XLIII)
worin M₁ für H, ein Alkalimetallkation oder ein Ammoniumkation steht, R₄₂ C₁-C₄-Alkyl und vorzugsweise H bedeutet, und R₄₁ der monovalente Rest eines chiralen ditertiären Diphosphins ist, wobei die CO-Gruppe direkt an ein C- oder N-Atom des Diphosphingerüsts, oder an ein O- oder N-Atom oder an ein C-Atom einer Brückengruppe des Diphosphingerüsts gebunden ist.

19. Verfahren gemäss Anspruch 1, **dadurch gekennzeichnet, dass** es sich bei den Hydrierkatalysatoren um Metallkomplexe der Formel XLIV, XLIVa und XLIVb handelt,
[X₇Me₂YZ] (XLIV),
[X₇Me₂Y]⁺A2⁻ (XLIVa),
[X₇Ru(II)X₈X₉] (XLIVb),
worin
Y für zwei Monoolefinliganden oder einen Dienliganden steht;
X₇ ein achirales oder chirales ditertiäres Diphosphin darstellt, das mit dem Metallatom Me₂ oder Ru einen 5- bis 7-gliedrigen Ring bildet;
Me₂ Ir(I) oder Rh(I) bedeutet;
Z -Cl, -Br oder -I darstellt; und
A₂ das Anion einer Sauerstoffsäure oder Komplexsäure ist,
X₈ und X₉ gleich oder verschieden sind und die Bedeutung von Z und A₂ haben, oder X₈ und X₉ für Allyl oder 2-Methylallyl stehen, oder X₈ die Bedeutung von Z oder A hat und X₉ für Hydrid steht.

## Claims

1. Method for producing tetrahydropterin and tetrahydropterin derivatives by hydrogenation of pterin and pterin derivatives with hydrogen in the presence of a hydrogenation catalyst, **characterised in that** hydrogenation is carried out in a polar reaction medium and d-8 metal complexes that are soluble in the reaction medium are used as hydrogenation catalysts containing tertiary phosphines, tertiary phosphanes as ligands, bidentate ligands having tertiary amino groups and tertiary phosphine groups, or bidentate ligands having two tertiary phosphine groups, with bidentate ligands forming a 5- to 10-membered ring with the metal atom.

2. Method according to claim 1, **characterised in that** the polar reaction medium is an aqueous or alcohol reaction medium.

3. Method according to claim 1, **characterised in that** as the pterin derivates folic acid, folic acid salts, folic acid esters, folic acid ester salts or dihydro forms thereof are used for the hydrogenation with hydrogen in the presence of a hydrogenation catalyst, hydrogenation is carried out at elevated pressure in the presence of metal complexes dissolved in the reaction medium and acting as hydrogenation catalysts, with the proviso that where folic acid, the carboxylic acid salts or dihydro forms thereof are used, the reaction medium is aqueous, and where folic acid esters, folic acid ester salts or drihydro forms thereof are used, the reaction medium is alcohol.

4. Method according to claim 1 for the asymmetrical hydrogenation of prochiral pterin derivatives with hydrogen in the presence of a hydrogenation catalyst, **characterised in that** hydrogenation is carried out in a polar reaction medium and metal complexes that are soluble in the reaction medium are used as hydrogenation catalysts, the metal complexes containing chiral ligands.

5. Method according to claim 4 for the asymmetrical hydrogenation of folic acid, folic acid salts, folic acid esters, folic acid ester salts or dihydro forms thereof as pterin derivatives, with hydrogen in the presence of a hydrogenation catalyst, **characterised in that** hydrogenation is carried out at elevated pressure in the presence of metal complexes dissolved in the reaction medium as hydrogenation catalysts, the metal complexes containing chiral ligands, with the proviso that where folic acid, carboxylic acid salts thereof or dihydro forms thereof are used, the reaction medium is aqueous, and where folic acid esters, folic acid ester salts or dihydro forms thereof are used, the reaction medium is alcohol.

6. Method according to claim 5, **characterised in that** the folic acid ester salts correspond to formula III and are in the form of their enantiomers or mixtures, 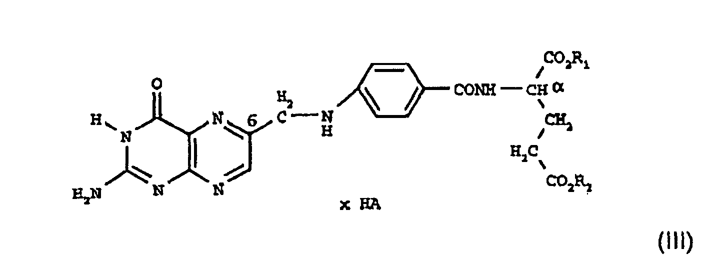 in which R₁ or R₂ is H, and R₂ or R₁, or both R₁ and R₂ independently of one another, represent a monovalent hydrocarbon radical or a heterohydrocarbon radical attached via a carbon atom, with heteroatoms selected from the group -O-, -S- and -N-,.
HA stands for a monobasic to tribasic inorganic or organic acid,
and x denotes an integer from 1 to 6 or a fractional number between 0 and 6.

7. Method according to claim 6, **characterised in that** the acid HA in formula III is unsubstituted or substituted phenyl sulphonic acid.

8. Method according to claim 1, **characterised in that** it is carried out at a hydrogen pressure of 1 to 500 bars.

9. Method according to claim 1, **characterised in that** the reaction temperature is 0 to 150°C.

10. Method according to claim 1, **characterised in that** the molar ratio of substrate to catalyst is 10 to 100,000.

11. Method according to claim 1, **characterised in that** the aqueous reaction medium is water or water in admixture with an organic solvent.

12. Method according to claim 1, **characterised in that** the alcohol reaction medium is an alcohol, or an alcohol in admixture with an organic solvent.

13. Method according to claim 1, **characterised in that** the metal complexes contain a d-8 metal, preferably iridium, rhodium or ruthenium.

14. Method according to claim 1, **characterised in that** the metal complex contains achiral or chiral ditertiary diphosphines as ligands.

15. Method according to claim 14, **characterised in that** the ditertiary disphosphines for an alcohol reaction medium are ones in which the phosphine groups are attached (a) to different carbon atoms of a carbon chain having 2 to 4 carbon atoms, or (b) directly or via a bridging group -CRₐR_{b}- in the orthopositions of a cyclopentadienyl ring or to a respective cyclopentadienyl of a ferrocenyl, Rₐ and R_{b} being the same or different and standing for H, C₁-C₈ alkyl, C₁-C₄ fluoroalkyl, C₅-C₆ cycloalkyl, phenyl, benzyl, or for phenyl or benzyl substituted with 1 to 3 C₁-C₄ alkyl or C₁ to C₄ alkoxy.

16. Method according to claim 15, **characterised in that** the diphosphines for an alcohol reaction medium correspond to formula IV
R₄R₅P-R₆-PR₇R₈ (IV),
in which
R₄, R₅, R₇ and R₈ independently of one another represent a hydrocarbon radical having 1 to 20 carbon atoms which are unsubstituted or substituted with halogen, C₁-C₆ alkyl, C₁-C₆ haloalkyl, C₁-C₆ alkoxy, C₁-C₆ haloalkoxy, (C₆H₅)₃Si, (C₁-C₁₂ alkyl)₃Si, -NH₂, -NH(C₁-C₁₂ alkyl), -NH(phenyl), -NH(benzyl), -N(C₁-C₁₂ alkyl)₂, - N(phenyl)₂, -N(benzyl)₂, morpholinyl, piperidinyl, pyrrolidinyl, piperazinyl, - ammonium-X₃⁻, -SO₃M₁, CO₂M1, -PO₃M₁, or -CO₂-C₁-C₆-alkyl, where M₁ represents an alkali metal or hydrogen, and X₃⁻ is the anion of a monobasic acid; or R₄ and R₅ and R₇ and R₈ respectively together denote tetramethylene, pentamethylene or 3-oxa-pentane-1,5-diyl which is unsubstituted or substituted with halogen, C₁-C₆ alkyl or C₁-C₆ alkoxy; and
R₆ is C₂-C₄ alkylene which is unsubstituted or substituted with C₁-C₆ alkyl, C₁-C₆ alkoxy, C₅ or C₆ cycloalkyl, phenyl, naphthyl or benzyl; 1,2 or 1,3 cycloalkylene, 1,2 or 1,3 cycloalkenylene, 1,2 or 1,3 bicycloalkylene or 1,2 or 1,3 bicycloalkenylene having 4 to 10 carbon atoms, either unsubstituted or substituted with C₁-C₆ alkyl, phenyl or benzyl; 1,2 or 1,3 cycloalkylene, 1,2 or 1,3 cycloalkenylene, 1,2 or 1,3 bicycloalkenylene or 1,2 or 1,3 bicycloalkenylene having 4 to 10 carbon atoms, attached at the 1 and/or 2 positions or at the 3 position of which is methylene or C₂-C₄ alkylidene, and either unsubstituted or substituted with C₁-C₆ alkyl , phenyl or benzyl; 1,4 butylene which is substituted in the 2,3 positions with R₉R₁₀C(O-)₂ and either unsubsituted in the 1 and/or 4 positions or substituted with C₁-C₆ alkyl, phenyl or benzyl, and with R₉ and R₁₀ independently of one another representing hydrogen, C₁-C₆ alkyl, phenyl or benzyl; 3,4 or 2,4 pyrrolidinylene or methylene-4-pyrrolidine-4-yl the N atom of which is substituted with hydrogen, C₁-C₁₂ alkyl, phenyl, benzyl, C₁-C₁₂ alkoxycarbonyl, C₁-C₈ acyl, C₁-C₁₂ alkylaminocarbonyl; or denotes 1,2-phenylene, 2-benzylene, 1,2-xylylene, 1,8-nathphylene, 2,2'-dinaphthylene or 2,2'-diphenylene, either unsubstituted or substituted with halogen, -OH, C₁-C₆ alkyl, C₁-C₆ alkoxy, phenyl, benzyl, phenyloxy or benzyloxy; or R₆ stands for a radical of the formulas 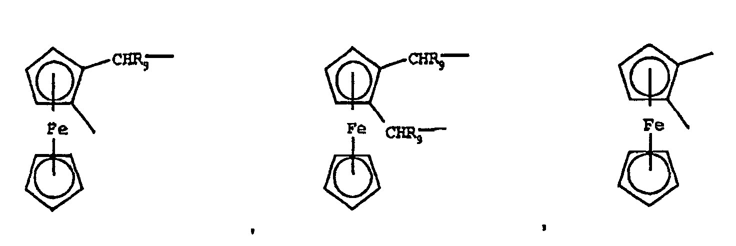 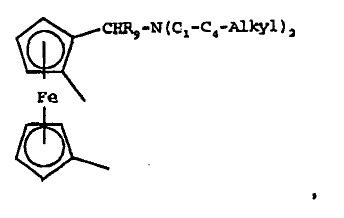 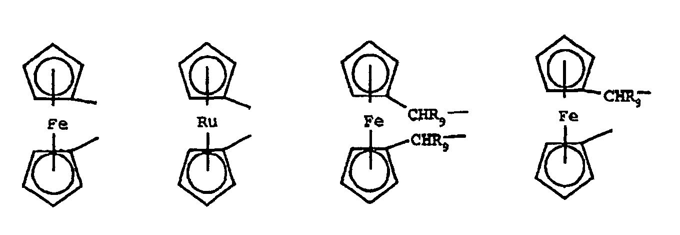 in which R₉ denotes hydrogen, C₁-C₈ alkyl, C₁-C₄ fluoroalkyl, unsubstituted phenyl or phenyl substituted with 1 to 3 F, Cl, Br, C₁-C4 alkyl, C₁-C₄ alkoxy or fluoromethyl.

17. Method according to claim 14, **characterised in that** diphosphines for an aqueous reaction medium are ones containing one or more water-solubilising polar substituents that are attached either directly or via a bridging group to substituents of the phosphine groups and/or to the skeleton of the diphosphine.

18. Method according to claim 17, **characterised in that** diphosphines for an aqueous reaction medium are ones of formula XLIII
(M₁O₂C-CH₂CH₂-O-CH₂)₃C-NR₄₂-CO-R₄₁ (XLIII)
in which M₁ stands for H, an alkali metal cation or an ammonium cation, R₄₂ denotes C₁-C₄ alkyl and preferably H, and R₄₁ is the monovalent radical of a chiral ditertiary diphosphine, with the CO group being attached directly to a C or N atom of the diphosphine skeleton, or to an O or N atom or to a C atom of a bridging group of the diphosphine skeleton.

19. Method according to claim 1, **characterised in that** the hydrogenation catalysts are metal complexes of formulas XLIV, XLIVa and XLIVb,
[X₇Me₂YZ] (XLIV),
[X₇Me₂Y]⁺A₂ (XLIVa),
[X₇Ru(II)X₈X₉] (XLIVb),
in which
Y stands for two monolefin ligands or a diene ligand;
X₇ represents an achiral or chiral ditertiary diphosphine which forms a 5 to 7 membered ring with the metal atom Me₂ or Ru;
Me₂ denotes Ir(I) or RH(I);
Z represents -Cl, -Br or -I; and
A₂ is the anion of an oxo acid or complex acid,
X₈ and X₉ are the same or different and have the meaning of Z and A₂,
or X₈ and X₉ stand for allyl or 2-methyl allyl, or X₈ has the meaning of Z or A and X₉ stands for hydride.

## Revendications

1. Procédé de préparation de tétrahydroptérine et de dérivés de tétrahydroptérine par hydrogénation de ptérine et de dérivés de ptérine avec de l'hydrogène en présence d'un catalyseur d'hydrogénation, **caractérisé en ce qu'**on met en oeuvre l'hydrogénation dans un milieu réactionnel polaire et qu'on utilise en tant que catalyseurs d'hydrogénation des complexes métalliques d-8 solubles dans le milieu réactionnel, qui contiennent des phosphines tertiaires, des phosphanes tertiaires en tant que ligands, des ligands bidentés ayant des groupes amino tertiaires et des groupes phosphine tertiaires, ou encore des ligands bidentés ayant deux groupes phosphine tertiaires, les ligands bidentés formant avec l'atome métallique un noyau à 5 à 10 chaînons.

2. Procédé selon la revendication 1, **caractérisé en ce que** le milieu réactionnel polaire est un milieu réactionnel aqueux ou alcoolique.

3. Procédé selon la revendication 1, **caractérisé en ce qu'**on utilise en tant que dérivés de la ptérine l'acide folique, les sels de l'acide folique, les esters de l'acide folique, les sels d'esters de l'acide folique ou leurs formes dihydratées pour assurer une hydrogénation avec l'hydrogène en présence d'un catalyseur d'hydrogénation, on met en oeuvre l'hydrogénation sous pression élevée en présence de complexes métalliques dissous dans le milieu réactionnel et servant de catalyseurs d'hydrogénation, à la condition que, si l'on utilise de l'acide folique, ses sels avec un acide carboxylique ou leurs formes dihydratées, on soit en présence d'un milieu aqueux, et que, si l'on utilise des esters de l'acide folique, des sels d'esters de l'acide folique ou leurs formes dihydratées, on soit en présence d'un milieu réactionnel alcoolique.

4. Procédé selon la revendication 1 pour l'hydrogénation asymétrique de dérivés de ptérine prochiraux à l'aide d'hydrogène en présence d'un catalyseur d'hydrogénation, **caractérisé en ce qu'**on met en oeuvre l'hydrogénation dans un milieu réactionnel polaire, et que l'on utilise en tant que catalyseurs d'hydrogénation des complexes métalliques solubles dans le milieu réactionnel, les complexes métalliques contenant des ligands chiraux.

5. Procédé selon la revendication 4 pour l'hydrogénation asymétrique de l'acide folique, de sels de l'acide folique, d'esters de l'acide folique, de sels d'esters de l'acide folique ou de leurs formes dihydratées en tant que dérivés de la ptérine, avec de l'hydrogène en présence d'un catalyseur d'hydrogénation, **caractérisé en ce qu'**on met en oeuvre l'hydrogénation sous pression élevée en présence de complexes métalliques dissous dans le milieu réactionnel et servant de catalyseurs d'hydrogénation, les complexes métalliques contenant des ligands chiraux, à la condition que, si l'on utilise de l'acide folique, ces sels avec un acide carboxylique ou leurs formes dihydratées, on soit en présence d'un milieu réactionnel aqueux, et que, si l'on utilise des esters de l'acide folique, des sels d'esters de l'acide folique ou leurs formes dihydratées, on soit en présence d'un milieu réactionnel alcoolique.

6. Procédé selon la revendication 5, **caractérisé en ce que** les sels d'esters de l'acide folique ont la formule III, et se présentent sous forme de leurs énantiomères ou de leurs mélanges, 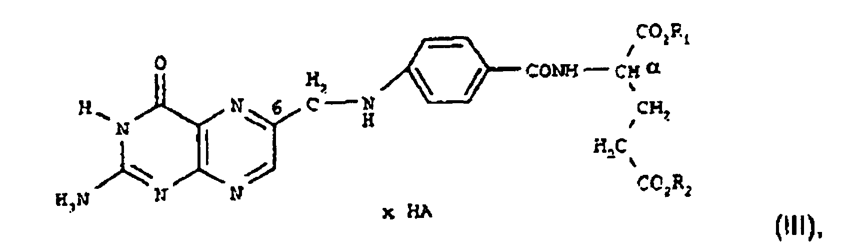 où R₁ ou R₂ est H, et R₁ ou R₂, ou les deux radicaux R₁ et R₂, représentent chacun indépendamment de l'autre un radical hydrocarboné monovalent ou un radical hétérohydrocarboné lié par l'intermédiaire d'un atome d'azote, contenant des hétéroatomes choisis dans l'ensemble comprenant -O-, -S- et -N-,
HA est un mono- à triacide inorganique ou organique,
et x est un nombre entier de 1 à 6, ou un nombre fractionnaire de 0 à 6.

7. Procédé selon la revendication 6, **caractérisé en ce que** l'acide HA est, dans la formule III, un acide phénylsulfonique non substitué ou substitué.

8. Procédé selon la revendication 1, **caractérisé en ce qu'**il est mis en oeuvre sous une pression d'hydrogène de 1 à 500 bar.

9. Procédé selon la revendication 1, **caractérisé en ce que** la température de réaction est de 0 à 150°C.

10. Procédé selon la revendication 1, **caractérisé en ce que** le rapport en moles du substrat au catalyseur est de 10 à 100 000.

11. Procédé selon la revendication 1, **caractérisé en ce que** le milieu réactionnel aqueux est l'eau, ou de l'eau en mélange avec un solvant organique.

12. Procédé selon la revendication 1, **caractérisé en ce que** le milieu réactionnel alcoolique est un alcool, ou un alcool en mélange avec un solvant organique.

13. Procédé selon la revendication 1, **caractérisé en ce que** les complexes métalliques contiennent un métal d-8, de préférence l'iridium, le rhodium ou le ruthénium.

14. Procédé selon la revendication 1, **caractérisé en ce que** le complexe métallique contient en tant que ligands des diphosphines ditertiaires achirales ou chirales.

15. Procédé selon la revendication 14, **caractérisé en ce que**, pour ce qui concerne les diphosphines ditertiaires destinées à un milieu réactionnel alcoolique, il s'agit de sels dans lesquels les groupes phosphine sont liés (a) à différents atomes de carbone d'une chaîne carbonée ayant de 2 à 4 atomes de carbone, ou (b) directement, ou par l'intermédiaire d'un groupe pontant -CRₐR_{b}- sur les positions ortho d'un noyau cyclopentadiényle, ou encore sont chacun liés à un groupe cyclopentadiényle d'un radical ferrocényle, Rₐ et R_{b} étant identiques ou différents et représentant chacun H, un groupe alkyle en C₁-C₈, fluoralkyle en C₁-C₄, cycloalkyle en C₅-C₈, phényle, benzyle, ou encore phényle ou benzyle une à trois fois substitué par des substituants alkyle en C₁-C₄ ou alcoxy en C₁-C₄.

16. Procédé selon la revendication 15, **caractérisé en ce que** les diphosphines destinées à un milieu réactionnel alcoolique ont la formule IV
R₄R₅P-R₆-PR₇R₈ (IV)
dans laquelle
R₄, R₅, R₇ et R₈ représentent chacun indépendamment des autres un radical hydrocarboné ayant de 1 à 20 atomes de carbone, qui sont non substitués, ou substitués par des substituants halogéno, alkyle en C₁-C₆, halogènalkyle en C₁-C₆, alcoxy en C₁-C₆, halogènalcoxy en C₁-C₆, (C₆H₅)₃Si, (alkyle en C₁-C₁₂)₃Si, -NH₂, -NH(alkyle en C₁-C₁₂), -NH(phényle), -NH(benzyle), -N(alkyle en C₁-C₁₂)₂, -N(phényle)₂, -N(benzyle)₂, morpholinyle, pipéridinyle, pyrrolidinyle, pipérazinyle, -ammonium-X₃⁻, -SO₃M₁, -CO₂M₁, -PO₃M₁ ou -CO₂-(alkyle en C₁-C₆), où M₁ est un métal alcalin ou un atome d'hydrogène, et X₃⁻ est l'anion d'un monoacide ; ou encore les paires de radicaux R₄ et R₅, ainsi que R₇ et R₈, représentent chacune un radical tétraméthylène, pentaméthylène ou 3-oxa-pentane-1,5-diyle, non substitué ou substitué par des substituants halogéno, alkyle en C₁-C₆ ou alcoxy en C₁-C₆ ; et
R₆ est un radical alkylène en C₂-C₄ non substitué ou substitué par des substituants alkyle en C₁-C₆, alcoxy en C₁-C₆, cycloalkyle en C₅ ou en C₆, phényle, naphtyle ou benzyle ; un radical 1,2- ou 1,3-cycloalkylène, 1,2- ou 1,3-cycloalcénylène, 1,2- ou 1,3-bicycloalkylène ou 1,2-ou 1,3-bicycloalcénylène ayant de 4 à 10 atomes de carbone, non substitué ou substitué par des substituants alkyle en C₁-C₆, phényle ou benzyle ; un radical 1, 2- ou 1,3-cycloalkylène, 1,2- ou 1,3-cycloalcénylène, 1,2- ou 1,3-bicycloalkylène ou 1,2- ou 1,3-bicycloalcénylène ayant de 4 à 10 atomes de carbone, non substitué ou substitué par des substituants alkyle en C₁-C₆, phényle ou benzyle, radicaux sur les positions 1 et/ou 2 desquels ou sur la position 3 desquels est lié un groupe méthylène ou alkylidène en C₂-C₄ ; un radical 1,4-butylène, substitué sur les positions 2,3 par R₉R₁₀C(O)₂, qui est non substitué sur les positions 1 et/ou 4, ou est substitué par des substituants alkyle en C₁-C₆, phényle ou benzyle, et R₉ et R₁₀ représentant chacun indépendamment de l'autre un atome d'hydrogène ou un groupe alkyle en C₁-C₆, phényle ou benzyle ; un radical 3,4- ou 2,4-pyrrolidinylène ou méthylène-4-pyrrolidine-4-yle dont l'atome d'azote est substitué par un ou plusieurs atomes d'hydrogène, ou groupes alkyle en C₁-C₁₂, phényle, benzyle, (alcoxy en C₁-C₁₂)carbonyle, acyle en C₁-C₈, (alkyle en C₁-C₁₂)aminocarbonyle ; ou encore un radical 1,2-phénylène, 2-benzylène, 1,2-xylylène, 1,8-naphtylène, 2,2'-dinaphtylène ou 2,2'-diphénylène non substitué ou substitué par des substi-tuants halogéno, -OH, alkyle en C₁-C₆, alcoxy en C₁-C₆, phényle, benzyle, phényloxy ou benzyloxy ;
ou encore R₆ est un radical ayant les formules 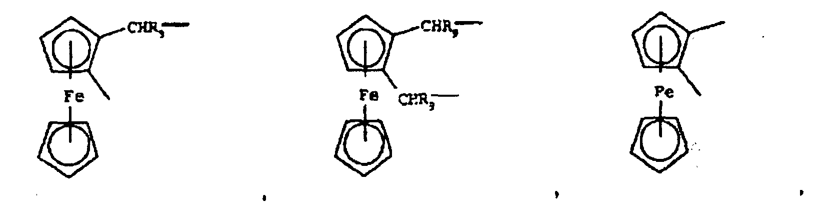 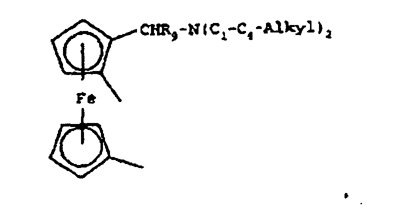 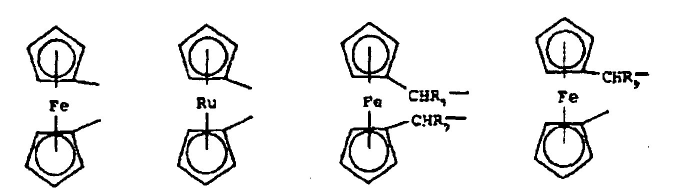 dans lesquels R₉ est un atome d'hydrogène ou un groupe alkyle en C₁-C₈, fluoralkyle en C₁-C₄, ou phényle non substitué ou substitué par un à trois substituants F, Cl, Br, alkyle en C₁-C₄, alcoxy en C₁-C₄ ou fluorométhyle.

17. Procédé selon la revendication 14, **caractérisé en ce que** les diphosphines destinées à un milieu réactionnel aqueux sont celles qui contiennent un ou plusieurs substituants polaires hydrosolubilisateurs, qui sont liés directement, ou par l'intermédiaire d'un groupe pontant, à des substituants des groupes phosphine et/ou au squelette de la diphosphine.

18. Procédé selon la revendication 17, **caractérisé en ce que** les diphosphines destinées à un milieu réactionnel aqueux sont celles qui ont la formule XLIII
(M₁O₂C-CH₂CH₂-O-CH₂)₃C-NR₄₂-CO-R₄₁ (XLIII)
dans laquelle M₁ est H ou un cation d'un métal alcalin ou un cation ammonium, R₄₂ est un groupe alkyle en C₁-C₄ et de préférence H, et R₄₁ est le résidu monovalent d'une diphosphine ditertiaire chirale, le groupe CO étant lié directement à un atome de carbone ou d'azote du squelette diphosphine, ou à un atome d'oxygène ou d'azote ou à un atome de carbone d'un groupe pontant du squelette diphosphine.

19. Procédé selon la revendication 1, **caractérisé en ce que**, pour ce qui concerne les catalyseurs d'hydrogénation, il s'agit de complexes métalliques ayant les formules XLIV, XLIVa et XLIVb
[X₇Me₂YZ] (XLIV)
[X₇Me₂Y]⁺A₂⁻ (XLIVa)
[X₇Ru(II)X₈X₉] (XLIVb)
dans lesquelles
Y représente deux ligands monooléfiniques ou un ligand diénique ;
X₇ est une diphosphine ditertiaire achirale ou chirale, qui avec l'atome métallique Me₂ ou Ru forment un noyau à 5 à 7 chaînons ;
Me₂ est Ir(I) ou Rh(I) ;
Z est -Cl, -Br ou -I ; et
A₂ est l'anion d'un oxacide ou d'un acide complexes ;
X₈ et X₉ sont identiques ou différents et ont la signification de Z et de A₂, ou encore X₈ et X₉ représentent chacun un groupe allyle ou 2-méthylallyle, ou encore X₈ a la signification de Z ou de A, et X₉ est un hydrure.
